# EUROPÄISCHE PATENTSCHRIFT

(11) **EP 1 113 021 B1**
(45) Veröffentlichungstag und Bekanntmachung des Hinweises auf die Patenterteilung: **09.08.2006**
(21) Anmeldenummer: 01104012.8
(22) Anmeldetag: 08.03.1995
(51) Int. Cl.: C07K 14/00, C07H 21/00

(54) **Polyamid-Oligonucleotid-Derivate, deren Herstellung und Verwendung**
Polyamide-oligonucleotide derivatives, their production and use
Dérivés des polyamide-oligonucleotides, leur production et utilisation

(30) Priorität: 14.03.1994 DE 4408528
(43) Veröffentlichungstag der Anmeldung: 04.07.2001
(62) Teilanmeldung aus: 95103332.3
(73) Patentinhaber: Sanofi-Aventis Deutschland GmbH, 65929 Frankfurt am Main (DE)
(72) Erfinder: Uhlmann, Eugen, Dr., 61479 Glashütten (DE); Breipohl, Gerhard, Dr., 60529 Frankfurt (DE)

(56) Entgegenhaltungen:
- EP-A- 0 552 766
- EP-A- 0 552 767
- WO-A-92/20702
- FREIER S.M. ET AL.: "Modified oligonucleotides: Hybridisation properties, pharmacokinetic properties and pharmacological activity." J. CELL BIOCHEM. SUPPL. - KEYSTONE SYMPOSIUM ON RIBOZYMES. (15-21 JAN 1995) MEETING ABSTRACT A6-017, Bd. 19a, Seite 205 XP002167075 BECKENRIDGE, CO, USA

## Beschreibung

Die vorliegende Erfindung betrifft neue Polyamid-Oligonucleotid-Derivate mit wertvollen physikalischen, biologischen und pharmakologischen Eigenschaften. Ihre Anwendung bezieht sich auf die Verwendung als Inhibitoren der Genexpression (Antisense Oligonucleotide, Ribozyme, Sense Oligonucleotide und Triplex Forming Oligonucleotide), als Sonden zum Nachweis von Nucleinsäuren und als Hilfsmittel in der Molekularbiologie.

Oligonucleotide finden in wachsendem Maße Anwendung als Inhibitoren der Genexpression (G. Zon, Pharmaceutical Research 5, 539 (1988); J. S. Cohen, Topics in Molecular and Structural Biology 12 (1989) Macmillan Press; C. Helene and J. J. Toulme, Biochimica et Biophysica Acta 1049, 99 (1990); E. Uhlmann and A. Peyman, Chemical Reviews 90, 543 (1990)). Antisense Oligonucleotide sind Nucleinsäure-Fragmente, deren Basensequenz komplementär ist zu einer zu inhibierenden mRNA. Diese Target-mRNA kann zellulären, viralen oder sonstigen pathogenen Ursprungs sein. Als zelluläre Target-Sequenzen kommen beispielsweise die von Rezeptoren, Zelladhäsionsproteinen, Enzymen, Immunmodulatoren, Cytokinen, Wachstumsfaktoren, lonenkanälen oder Onkogenen in Frage. Die Inhibition der Virus Vermehrung mit Hilfe von Antisense Oligonucleotiden wurde beispielsweise für HBV (Hepatitis B Virus), HSV-1 und -2 (Herpes Simplex Virus Typ I und II), HIV (Human Immunodeficiency Virus) und Influenza-Viren beschrieben. Dabei setzt man Oligonucleotide ein, die zur viralen Nucleinsäure komplementär sind. Sense Oligonucleotide sind dagegen in ihrer Sequenz so konzipiert, daß sie beispielsweise Nucleinsäure-bindende Proteine oder Nucleinsäure-prozessierende Enzyme binden ("einfangen") und so deren biologische Aktivität inhibieren (C. Helene and J. J. Toulme, Biochimica et Biophysica Acta 1049, 99 (1990)). Als virale Targets sind hier beispielsweise die Reverse Transkriptase, DNA-Polymerase und Transaktivator-Proteine zu nennen. Triplex Forming Oligonucleotide haben im allgemeinen die DNA als Target und bilden nach Bindung an diese eine tripelhelicale Struktur aus. Während mit Hilfe der Antisense Oligonucleotide im allgemeinen die Prozessierung (Splicing etc.) der mRNA oder deren Translation in das Protein gehemmt wird, hemmen Triplex Forming Oligonucleotide die Transcription oder Replikation der DNA (C. Helene und J.J. Toulme; Biochim. Biophys. Acta 1049 (1990) 99-125; E. Uhlmann and A. Peyman, Chemical Reviews 90, 543 (1990)). Es ist aber auch möglich, einzelsträngige Nucleinsäuren in einer ersten Hybridisierung mit einem Antisense Oligonucleotid unter Ausbildung eines Doppelstranges zu binden, der dann in einer zweiten Hybridisierung mit einem Triplex Forming Oligonucleotid eine Triplex-Struktur ausbildet. Die Antisense und Triplex Bindungsregionen können dabei entweder in zwei separaten Oligonucleotiden oder aber in einem Oligonucleotid beherbergt sein. Eine weitere Anwendung synthetischer Oligonucleotide sind die sogenannten Ribozyme, welche die Target-RNA infolge ihrer Ribonuclease-Aktivität zerstören (J.J. Rossi and N. Sarver, TIBTECH (1990) 8, 179; Castanetto et al., Critical Rev. Eukar. Gene Expr. (1992) 2, 331).
Die erfindungsgemäßen Verbindungen können auch im Sinne von Aptameren in der Therapie eingesetzt werden. Aptamere sind oligomere Nucleinsäuren oder deren Analoga, welche mit hoher Affinität an Proteine binden. Das Auffinden der Aptameren erfolgt durch in vitro Selektion aus einem Zufallsgemisch (Famulok und Szostak (1992) Angew. Chem. 104, 1001-1011) und wurde für ein Thrombin-bindendes Aptamer erfolgreich durchgeführt (Bock et al. (1992) Nature 355, 564-566). Man kann dabei so verfahren, daß die Basenfolge des Aptamers durch Screening eines Oligonucleotid-Gemisches bestimmt wird und diese Basensequenz dann auf Polyamid-Oligonucleotid-Analoga übertragen wird. Eine andere Möglichkeit besteht darin, daß die bindende Region des Aptamers zur Erleichterung der Identifikation durch einen separaten nichtbindenden Teil des Moleküls codiert wird (Brenner und Lerner (1992) PNAS 89, 5381-5383).

In der DNA-Diagnostik werden Nucleinsäure-Fragmente mit geeigneter Markierung als sogennante DNA-Sonden oder DNA-Probes für die spezifische Hybridisierung an eine nachzuweisende Nucleinsäure eingesetzt. Die spezifische Ausbildung des neuen Doppelstranges wird dabei mit Hilfe der Markierung, die vorzugsweise nicht radioaktiv ist, verfolgt. Auf diese Weise lassen sich genetische, maligne, virale oder durch andere pathogene verursachte Krankheiten nachweisen.
Für die meisten genannten Anwendungen sind Oligonucleotide in ihrer natürlich vorkommenden Form wenig oder völlig ungeeignet. Sie müssen chemisch so modifiziert werden, daß sie den speziellen Anforderungen gerecht werden. Damit Oligonucleotide in biologischen Systemen, beispielsweise zur Inhibition der Virus-Vermehrung eingesetzt werden können, müssen sie folgende Voraussetzungen erfüllen:
1. Sie müssen unter in vivo Bedingungen, also sowohl im Serum als auch intrazellulär, eine ausreichend große Stabilität aufweisen.
2. Sie müssen so beschaffen sein, das sie die Zell- und Nucleus-Membran passieren können.
3. Sie müssen unter physiologischen Bedingungen in Basen-spezifischer Weise an ihre Target-Nucleinsäure binden, um den inhibitorischen Effekt zu entfalten.

Für DNA-Sonden sind die Punkte 1 bis 3 keine Voraussetzung; jedoch müssen diese Oligonucleotide so derivatisiert sein, daß ein Nachweis, beispielsweise mittels Fluoreszenz, Chemilumineszenz, Kolorimetrie oder spezifischer Färbung, möglich ist (Beck und Köster, Anal. Chem. 62, 2258 (1990)).
Die chemische Veränderung der Oligonucleotide erfolgt meistens in der Weise, daß Phosphatrückgrat, Ribose-Einheit oder die Nucleobasen entsprechend verändert werden (J. S. Cohen, Topics in Molecular and Structural Biology 12 (1989) Macmillan Press; E. Uhlmann and A. Peyman, Chemical Reviews 90, 543 (1990)). Eine weitere häufig genutzte Methode ist die Herstellung von Oligonucleotid-5'-Konjugaten durch Umsetzung der 5'-Hydroxy-Gruppe mit entsprechenden Phosphorylierungs-Reagenzien. Wenn dagegen alle Internucleotid-Phosphat-Reste verändert werden, verändern sich die Eigenschaften der Oligonucleotide oft drastisch. Beispielsweise ist die Löslichkeit von Methylphosphonaten in wässrigem Medium stark vermindert, während All-Phosphorothioat-Oligonucleotide oft sequenzunspezifisch wirken.

Kürzlich wurden Polyamid-Nucleinsäure-Derivate beschrieben (Michael Egholm, Peter E. Nielsen, Rolf H. Berg and Ole Buchardt, Science 1991, 254, 1497-1500; WO 92/20702; M. Egholm et al. Nature (1993) 365, 566-568; P. Nielsen, (1994) Bioconjugate Chem. 5, 3-7), die mit höherer Affinität als natürliche Oligonucleotide an komplementäre Zielsequenzen (DNA oder RNA) binden. Diese sogenannten Peptide bzw. Polyamide Nucleic Acids (PNA) sind DNA-analoge Verbindungen, in denen das Deoxyribose-Phosphat-Gerüst durch ein Polyamid-Oligomer ersetzt wurde. Diese Verbindungen haben den Vorteil gegenüber den natürlichen Oligonucleotiden, daß sie im Serum sehr stabil sind. Sie haben aber andererseits folgende nachteilige Eigenschaften:
(1) Sie werden nicht bzw. nur in nicht nachweisbarer Menge in Zellen aufgenommen. Da Antisense oder Triplex-Forming Oligonucleotide ihre Aktivität aber nur in der Zelle entfalten können, sind die PNA's als solche ungeeignet zur Inhibition der Genexpression in vivo.
(2) Die PNA's tendieren in wässriger Lösung, also auch unter physiologischen Bedingungen zur Aggregation. Sie sind daher in wässrigem Puffer schlecht löslich und stehen nicht für die Hybridisierung an komplementäre Sequenzen zur Verfügung.
(3) Die PNA's haben zudem eine hohe Affinität zu verschiedenen Materialien wie ® Sephadex (Fa. Pharmacia) oder ® Bond Elut (Fa. Varian), die bei der Aufreinigung der Oligomeren Verwendung finden, so daß die PNA's oft nur in schlechten Ausbeuten zu isolieren sind.
(4) Ein weiterer gravierender Nachteil der PNA's besteht darin, daß sie nicht in einer eindeutigen Orientierung an komplementäre Nucleinsäuren binden. Deshalb ist die Sequenzspezifität gegenüber den natürlichen Oligonucleotiden reduziert. Während natürliche Nucleinsäuren an komplementäre Nucleinsäuren im allgemeinen in antiparalleler Orientierung hybridisieren, können PNA's sowohl in antiparalleler als auch in paralleler Orientierung binden.
(5) In WO 92/20702 ist ein Oligonucleotid-PNA-Konjugat (T)₇(5'-L-N)(t)₆-Ala erwähnt (Fig. 25; substitute sheet), wobei (T)₇ ein natürliches Heptathymidylat Oligonucleotid bedeutet, das über sein 5'-O-Phosphat und 4-Hydroxybuttersäure (L) an die primäre Aminofunktion (N) eines PNA-Hexathymidylats (t)₇ und Alanin (Ala) gebunden ist. Es wurden weder Synthese dieser Verbindung noch irgendwelche Eigenschaften beschrieben.
(6) PNA's zeigen in Zellkulturexperimenten im *µ*molaren Bereich stark cytotoxische Eigenschaften.

Die Orientierung der basenpaarenden Nucleinsäurestränge ist wie folgt definiert: (vgl. Egholm et al.; Nature 365 (1993) 566-56

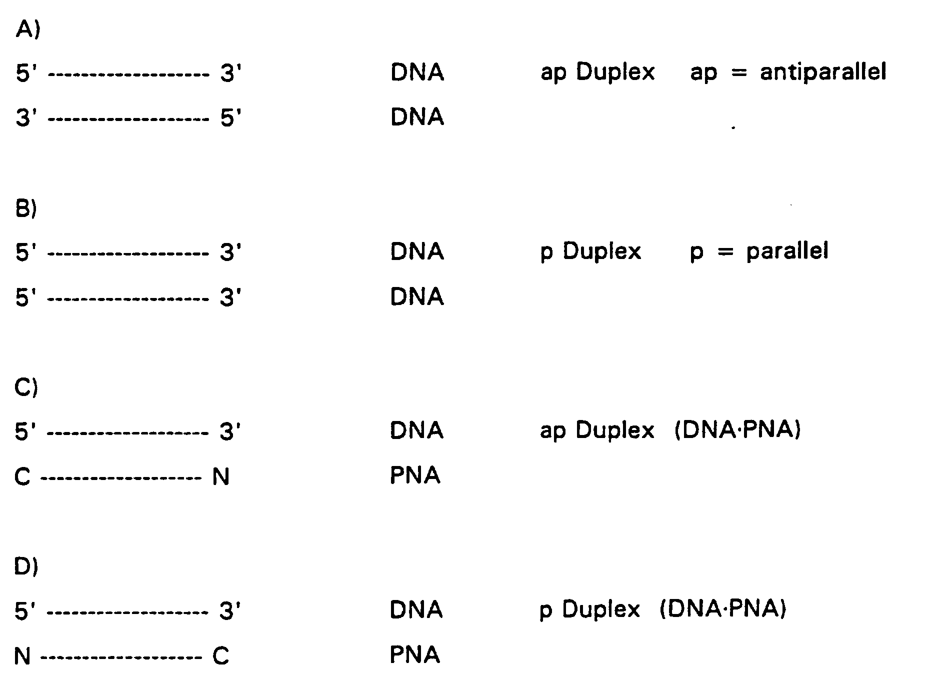

8).

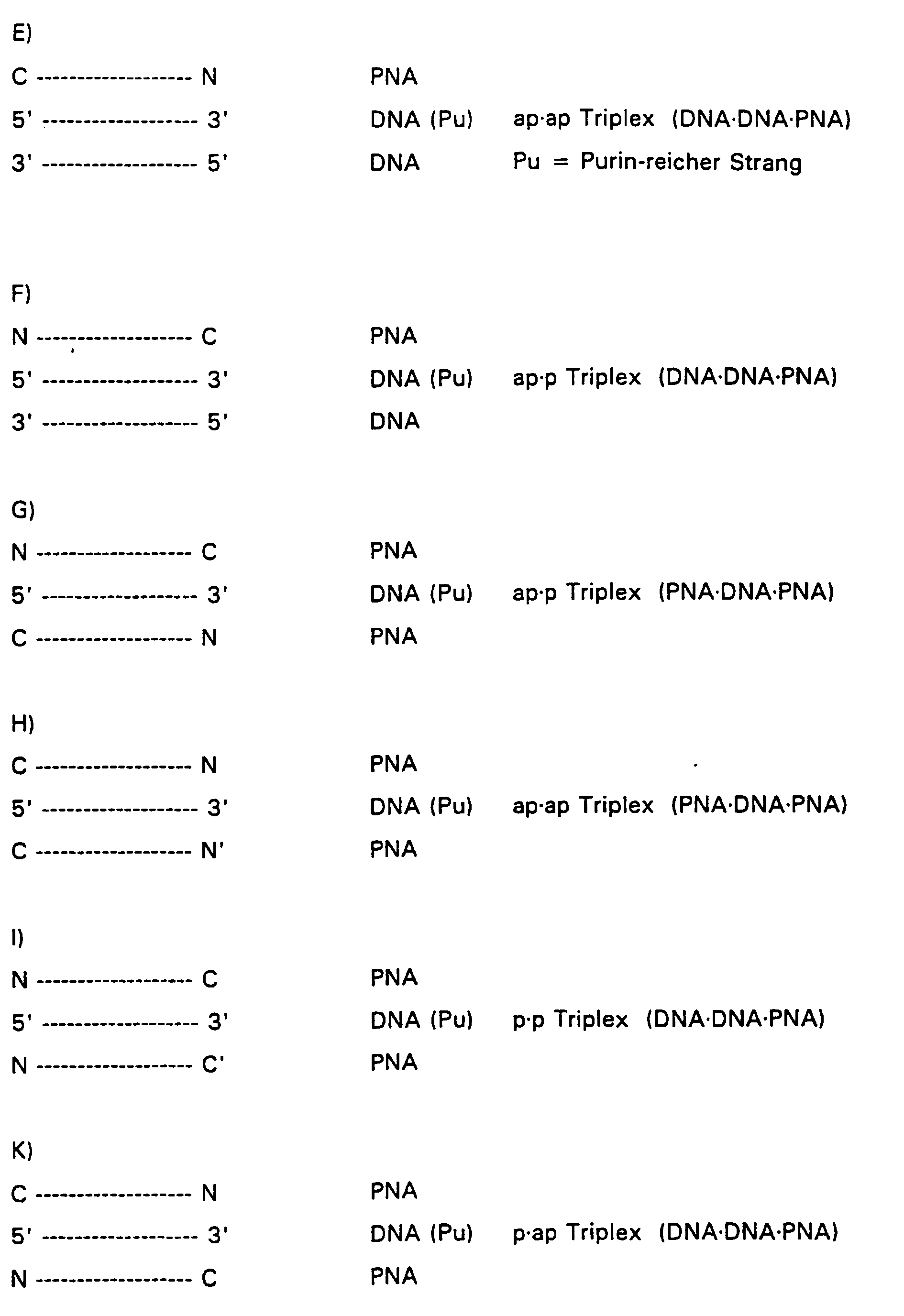

wobei
- 5': das 5'-Ende eines Oligonucleotids,
- 3': das 3'-Ende eines Oligonucleotids,
- N: den Aminoterminus eines PNA's
- C: den Carboxyterminus eines PNA's bedeuten.

Die Fälle A) - D) sind beispielhaft für die prinzipiell möglichen Orientierungsarten der Antisense-Oligomeren.Die Fälle E) - F) zeigen Möglichkeiten der Triplexbildung an einzelsträngigen oder doppelsträngigen Nucleinsäuren. Dabei können zwei der PNA- bzw. DNA-Einzelstränge miteinander verknüpft sein. Beispielsweise kann in E) der N-Terminus des PNA's mit dem 5'-Ende der DNA oder in F) der C-Terminus des PNA mit dem 5'-Ende der DNA verknüpft sein.

Die Aufgabe der Erfindung bestand deshalb darin, Polyamid-Oligonucleotid-Derivate herzustellen, in denen oben genannten Nachteile eliminiert sind.

Gegenstand der Erfindung sind Polyamid-Oligonucleotid-Derivate der Formel Ib 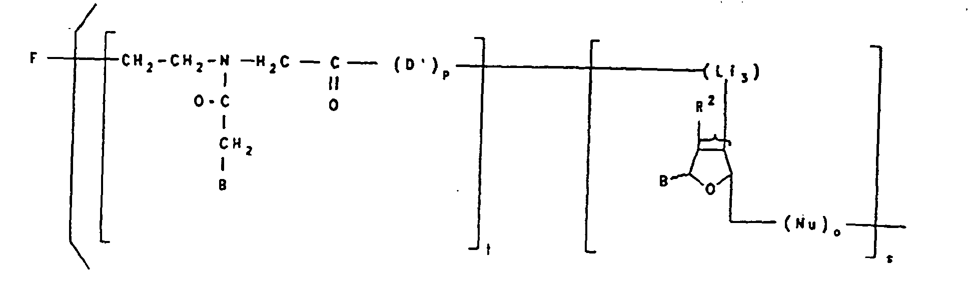 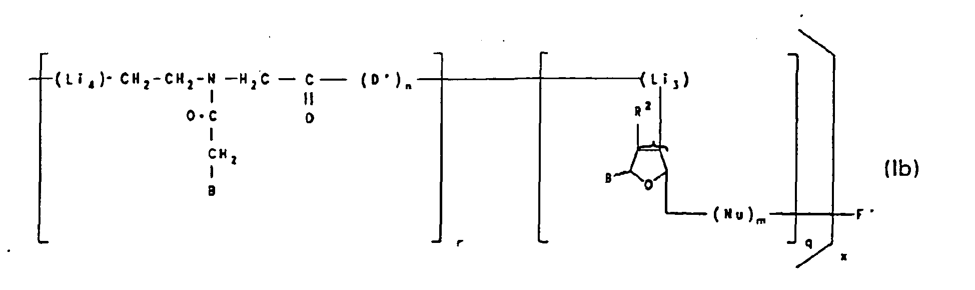 worin
- x 1: bedeutet, wobei
r = s = 1 undgleichzeitig q = t = Null
oder q=r=1 und s=t=Null
oder q=r=s und t =Null
sind;
- R²: Wasserstoff, Hydroxy, C₁-C₁₈-Alkoxy, bevorzugt C₁-C₆-Alkoxy, Halogen, wie F oder Cl, bevorzugt F, Azido oder Amino bedeutet;
- B: unabhängig voneinander für eine in der Nucleotidchemie übliche Base, beispielsweise für natürliche Basen wie Adenin, Cytosin, Thymin, Guanin, Uracil, Inosin oder unnatürliche Basen wie beispielsweise Purin, 2.6-Diaminopurin, 7-Deazaadenin, 7-Deazaguanin, N⁴N⁴-Ethanocytosin, N⁶N⁶⁻Ethano-2.6-diaminopurin, Pseudoisocytosin, 5-Methylcytosin, 5-Fluoruracil, 5-(C₃-C₆)-Alkinyluracil, 5-(C₃-C₆)-Alkinylcytosin oder deren Prodrugformen steht,
und die "geschweifte Klammer" andeutet, daß sich R² und der benachbarte Substituent in 2'- und 3'-Stellung oder auch umgekehrt in 3'- und 2'-Stellung befinden können; und wobei die Polyamid-Struktur mindestens eine Nukleobase enthält, die von Thymin verschieden ist;
- Nu: für einen Rest der Formeln IIa oder llb steht 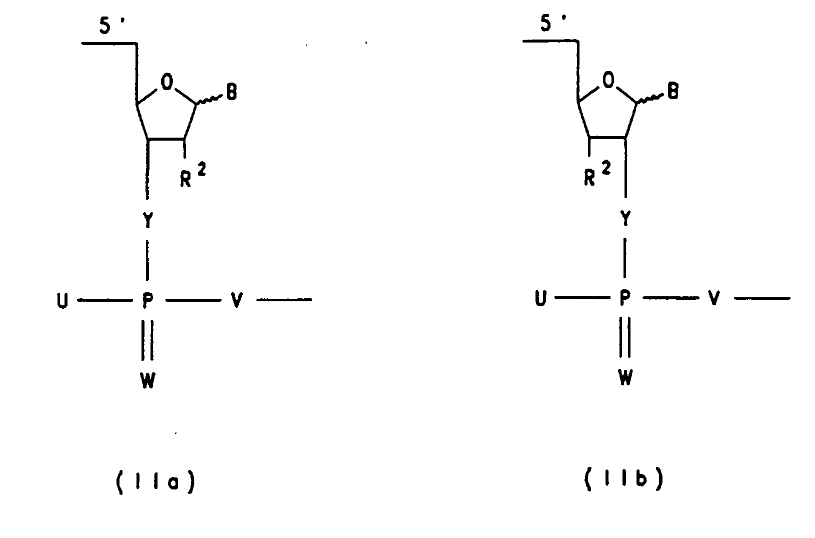 worin
R² und B wie oben definiert sind;
U Hydroxy, Mercapto, C₁-C₁₈-Alkyl, bevorzugt C₁-C₈-Alkyl, C₁-C₁₈₋Alkoxy, bevorzugt C₁-C₈-Alkoxy, C₆-C₂₀-Aryl, bevorzugt C₆-C₁₂₋Aryl, C₆-C₁₄ Aryl-C₁-C₈-alkyl, bevorzugt C₆-Aryl-C₁-C₄-alkyl, NHR³ oder NR³R⁴ bedeutet und
- R³: C₁-C₁₈-Alkyl oder C₁-C₄-Alkoxy-C₁-C₄-alkyl ist, vorzugsweise C₁-C₈-Alkyl oder C₁-C₄-Alkoxy-C₁-C₄-alkyl, besonders bevorzugt C₁-C₄-Alkyl oder Methoxyethyl und
- R⁴: C₁-C₁₈-Alkyl, vorzugsweise C₁-C₈-Alkyl und besonders bevorzugt C₁-C₄₋Alkyl, ist oder
- R³ und R⁴: zusammen mit dem sie tragenden Stickstoffatom einen 5-6-gliedrigen heterozyklischen Ring bedeutet, der zusätzlich ein weiteres Heteroatom aus der Reihe O, S, N enthalten kann, wie beispielsweise Morpholin;
- V: Oxy, Sulfanidyl oder lmino bedeutet;
- W: Oxo oder Thioxo bedeutet;
- Y: Oxy, Sulfanidyl, Methylen oder Imino bedeutet;
- m: = Null bis 20;
- o =: Null bis 20;
- D': einen Rest der Formel IV bedeutet 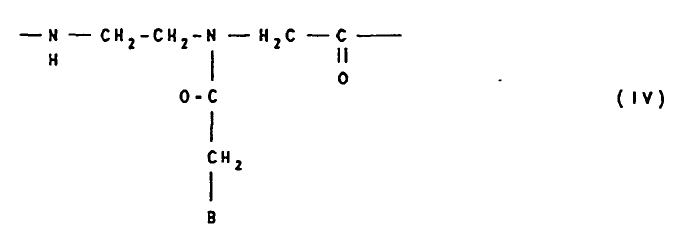 worin B wie oben definiert ist;
- n =: Null bis 20;
- p =: Null bis 20;
Li₃ und Li₄ unabhängig voneinander jeweils eine Struktur der Formel V

[(V')-(G)-(G')]_{ε} (V)

ist, wobei unabhängig voneinander
ε = 1 bis 5, bevorzugt 1 - 2 ist,
V' Sauerstoff, NH, eine Bindung oder einen Rest der Formel VI 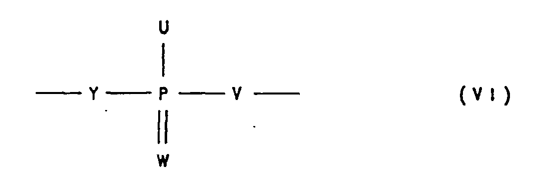 bedeuten,
worin U, V, W und Y wie oben definiert sind;
- G: C₁-C₁₂-Alkandiyl, bevorzugt C₁-C₆-Alkandiyl, wobei Alkandiyl gegebenenfalls durch Halogen, bevorzugt F oder Chlor, Amino, Hydroxy, C₁-C₁₈-Alkyl, bevorzugt C₁-C₆-Alkyl, C₁-C₁₈-Alkoxy, bevorzugt C₁-C₆₋Alkoxy, C₆-C₁₄-Aryl, bevorzugt C₆-Aryl, oder C₆-C₁₄-Aryl-C₁-C₁₈-Alkyl, bevorzugt C₆-Aryl-C₁-C₄-Alkyl substituiert sein kann; C₆-C₁₄-Aryl-di-C₁-C₁₂₋Alkandiyl, bevorzugt C₆-Aryl-di-C₁-C₄-Alkandiyl, oder einer Gruppe der Formel (CH₂CH₂O)_{δ}CH₂CH₂, worin ö gleich 1 bis 11, bevorzugt 1 bis 7 sein kann; oder für eine Bindung stehen kann; und
- G': Oxy, Sulfanidyl, Imino, -C(O)-, -C(O)NH-, eine Bindung oder einen Rest der Formel VI bedeuten, worin U, V, W und Y wie oben definiert sind; und
- F und F': über eine Bindung verknüpft sind (cyclische Verbindungen) und/oder
- F: für R⁰ - (A)ₖ - V - und
- F': in Formel Ib für V¹ - (A), - R¹ stehen,
wobei
R⁰ Wasserstoff, C₁-C₁₈-Alkanoyl, bevorzugt C₈-C₁₈-Alkanoyl, C₁-C₁₈₋Alkoxycarbonyl, C₃-C₈-Cycloalkanoyl, C₇-C₁₅-Aroyl, C₃-C₁₃-Heteroaroyl oder eine Gruppe bedeuten, die die intrazelluläre Aufnahme des Oligomers begünstigt oder als Markierung einer DNA-Sonde dient oder bei der Hybridisierung des Oligomers an die target-Nucleinsäure diese unter Bindung, Quervernetzung oder Spaltung angreift; oder,
falls k = Null ist, R⁰ Wasserstoff ist oder zusammen mit V für einen Rest der Formel VII 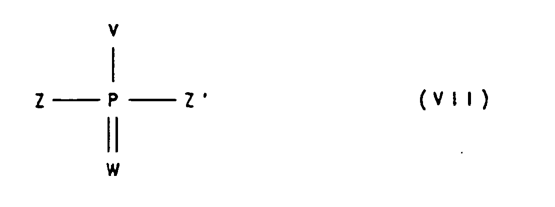
steht, worin
Z und Z' unabhängig voneinander Hydroxy, Mercapto, C₁-C₂₂-Alkoxy, bevorzugt C₁₂-C₁₈-Alkoxy, C₁-C₁₈-Alkyl, bevorzugt C₁₂-C₁₈-Alkyl, C₆-C₂₀₋Aryl, bevorzugt C₆-C₁₆-Aryl, C₆-C₁₄-Aryl-C₁-C₁₈-Alkyl, bevorzugt C₆-Aryl-C₁-C₄-Alkyl, C₁-C₂₂-Alkylthio, bevorzugt C₁₂-C₁₈-Alkylthio, NHR³, NR³R⁴, oder eine Gruppe bedeuten, die die intrazelluläre Aufnahme des Oligomers begünstigt oder als Markierung einer DNA-Sonde dient oder bei der Hybridisierung des Oligomers an die target-Nucleinsäure diese unter Bindung, Quervernetzung oder Spaltung angreift, und worin
R³, R⁴, V und W wie oben definiert sind;
- R¹: Wasserstoff oder Q⁰
wobei R¹ immer nur dann Wasserstoff ist,
wenn gleichzeitig I = Null und
in Formel Ib q = 1 oder q = r = Null und in F' = V¹ - (A), - R¹ mit V¹ = V bedeuten,
- A: den Rest einer natürlichen oder unnatürlichen Aminosäure, bevorzugt aus der Reihe Glycin, Leucin, Histidin, Phenylalanin, Cystein, Lysin, Arginin, Asparaginsäure, Glutaminsäure, Prolin, Tetrahydroisochinolin-3-carbonsäure, Octahydroindol-2-carbonsäure, N-(2-Aminoethyl)glycin bedeutet
- Q⁰: Hydroxy, OR', NH₂, NHR" bedeutet mit
R' = C₁-C₁₈-Alkyl, bevorzugt C₁₂-C₁₈-Alkyl und
R" = C₁-C₁₈-Alkyl, bevorzugt C₁₂-C₁₈-Alkyl, C₁-C₁₈-Aminoalkyl, bevorzugt C₁₂-C₁₈-Aminoalkyl, C₁-C₁₈-Hydroxyalkyl, bevorzugt C₁₂₋C₁₈-Hydroxyalkyl;
- V: wie oben definiert ist;
- V¹: eine Bindung oder V ist, wobei in F' nur in Formel lb mit q = Null
und r = 1 V¹ immer für eine Bindung steht;
- k: Null bis 10 ist;
- l: Null bis 10 ist;
mit der Maßgabe, daß
a) falls in der Verbindung der Formel Ib t = Null und s = 1 sind, steht Li₃ für eine Bindung;
b) falls in der Verbindung der Formel Ib s = t = Null ist, steht Li₄ für eine Bindung;
wobei jedes Nucleotid in seiner D- bzw. L-Konfiguration vorliegen kann und sich die Base in α- oder β-Stellung befinden kann.

Besonders bevorzugt sind Verbindungen der Formel lb, in denen sich die Base am Zucker in β-Stellung befindet,
x = 1 ist und
q=r=1, s=t=Null oder
r=s=1, q=t=Null oder
q=r=s=1, t=Null oder
r=s=t=1, q=Null sind.

Insbesondere bevorzugt sind Oligomere der Formel Ib, worin V', V, Y und W die Bedeutung von Thio, Oxy, Oxo oder Hydroxy haben; ganz besonders bevorzugt sind diese, falls zusätzlich R² gleich Wasserstoff ist.

Insbesondere bevorzugt sind auch Oligomere der Formel Ib mit ε = 1, in denen
- Li₄: a) eine Verbindung der Formel V, in der V' = Sauerstoff oder eine Verbindung der Formel VI, G = C₁-C₁₀-Alkylen, G' =-CONH-
b) eine Verbindung der Formel V, in der G, V' eine Bindung und G' eine Verbindung der Formel VI ist mit bevorzugt
   U = V = W = Y = Sauerstoff oder U = W = Y = Sauerstoff und V = Imino
- Li₃: a) eine Verbindung der Formel V mit V' = Imino, G = C₁-C₁₀-Alkylen und G' = Verbindung der Formel VI
b) eine Verbindung der Formel V mit V' = Imino, G und G' = Bindung
c) eine Verbindung der Formel V mit V' = Imino, G = C₁-C₁₀-Alkylen und G' = V mit bevorzugt U = V = W = Y = Sauerstoff.

Ganz besonders bevorzugt sind Oligomere der Formel Ib, worin V', V, Y und W die Bedeutung von Thio, Oxy, Oxo oder Hydroxy haben, R² gleich Wasserstoff ist, Li₁ die Bedeutung von -V'-[CH₂]ₙC(O)NH- mit V' = Verbindung der Formel Vl mit U =V=W=Y=Sauerstoff oder Li₂ die Bedeutung von -HN-[CH₂]ₙ(G')- haben, wobei n = 2 bis 5 und G' gleich der Formel VI mit U, V, W und Y = Sauerstoff ist.

Außerdem werden Oligomere der Formel Ib bevorzugt, worin V', V, Y und W die Bedeutung von Thio, Oxy, Oxo oder Hydroxy haben, R² gleich Wasserstoff ist, Li₁, die Bedeutung von -O-[CH₂]ₙC(O)NH- oder Li₂ die Bedeutung von -HN-[CH₂]ₙ(G')- haben, wobei n= 2 bis 5 und G' gleich der Formel VI mit U,V,W und Y=Sauerstoff ist und q=Null und r=s=t=1 sind.

Bevorzugt sind außerdem Oligomere der Formel b, worin die geschweifte Klammer bedeutet, daß sich R² in 3' Stellung (siehe Formel llb) befindet. Die bevorzugte Base ist hierbei Adenin.

Die Erfindung ist nicht auf α- und β-D- bzw. L-Ribofuranoside, α- und β-D- bzw. L- Desoxyribofuranoside und entsprechende carbocyclische Fünfringanaloga beschränkt, sondern gilt auch für Oligonucleotidanaloga, die aus anderen Zucker-Bausteinen aufgebaut sind, beispielsweise ringerweiterte und ringverengte Zucker, acyclische, ringverbrückte oder geeignete andersartige Zucker-Derivate. Die Erfindung ist ferner nicht auf die in Formel I beispielhaft aufgeführten Derivate des Phosphat-Restes beschränkt, sondern bezieht sich auch auf die bekannten Dephospho-Derivate.

Der Oligonucleotid-Teil (DNA in Formel l) kann also in vielfältiger Weise von der natürlichen Struktur abgewandelt sein. Solche Modifikationen, die nach an sich bekannten Methoden eingefürt werden, sind beispielsweise:

### a) Modifikationen der Phosphatbrücke

Beispielhaft seien genannt: Phosphorothioate, Phosphorodithioate, Methylphosphonate, Phosphoramidate, Boranophosphate, Phosphatmethylester, Phosphatethylester, Phenylphosphonate. Bevorzugte Modifikationen der Phosphatbrücke sind Phosphorothioate, Phosphorodithioate und Methylphosphonate.

### b) Ersatz der Phosphatbrücke

Beispielhaft seien genannt: Ersatz durch Formacetal, 3'-Thioformacetal, Methylhydroxylamin, Oxim, Methylendimethylhydrazo, Dimethylensulfon, Silylgruppen. Bevorzugte ist der Ersatz durch Formacetale und 3'-Thioformacetale.

### c) Modifikationen des Zuckers

Beispielhaft seien genannt: α-anomere Zucker, 2'-O-Methylribose, 2'-O-Butylribose, 2'-O-Allylribose, 2'-Fluoro-2'-desoxyribose, 2'-Amino-2'-desoxyribose, α-Arabinofuranose, Carbocyclische Zuckeranaloge. Bevorzugte Modifikation ist die durch 2'-O-Methylribose und 2'-O-n-Butylribose.

### d) Modifikationen der Basen welche die Spezifität der Watson-Crick

Basenpaarung nicht verändern
Beispielhaft seien genannt: 5-Propinyl-2'-desoxyuridin, 5-Propinyl-2'-desoxycytidin, 5-Hexinyl-2'-desoxyuridin, 5-Hexinyl-2'-desoxycytidin, 5-Fluoro-2'-desoxycytidin, 5-Fluor-2'-desoxyuridin, 5-Hydroxymethyl-2'-desoxyuridin, 5-Methyl-2'-desoxycytidin, 5-Brom-2'-desoxycytidin. Bevorzugte Modifikationen sind 5-Propinyl-2'-desoxyuridin, 5-Hexinyl-2'-desoxyuridin, 5-Hexinyl-2'-desoxycytidin und 5-Propinyl-2'-desoxycytidin.

### e) 3'-3'- und 5'-5'-Inversionen [z.B. M. Koga et al., J. Org. Chem. 56 (1991) 3757]

### f) 5'- und 3'-Phosphate, sowie 5'- und 3'-Thiophosphate.

Beispielhaft für Gruppen, die die intrazelluläre Aufnahme begünstigen, sind verschiedene lipophile Reste wie -O-(CH₂)ₓ-CH₃, worin x eine ganze Zahl von 6 bis 18 bedeutet, -O-(CH₂)ₙ-CH=CH-(CH₂)ₘ-CH₃, worin n und m unabhängig voneinander eine ganze Zahl von 6 bis 12 bedeuten, -O-(CH₂CH₂O)₄-(CH₂)₉-CH₃, -O-(CH₂CH₂O)₈- (CH₂)₁₃-CH₃ und -O-(CH₂CH₂O)₇-(CH₂)₁₅-CH₃, aber auch Steroid-Reste wie Cholesteryl oder Vitamin-Reste wie Vitamin E, Vitamin A oder Vitamin D und andere Konjugate, die natürliche Carriersysteme ausnutzen wie Gallensäure, Folsäure, 2-(N-Alkyl, N-Alkoxy)-Aminoanthrachinon und Konjugate der Mannose und Peptide der entsprechenden Rezeptoren, die zur rezeptorvermittelten Endozytose der Oligonucleotide führen wie EGF (Epidermal Growth Factor), Bradykinin und PDGF (Platelet Derived Growth Factor). Unter Markierungs-Gruppen sind fluoreszlerende Gruppen beispielsweise von Dansyl-(=N-Dimethyl-1-aminonaphthyl-5-sulfonyl-), Fluorescein- oder Coumarin-Derivaten oder chemilumineszierende Gruppen beispielsweise von Acridin-Derivaten zu verstehen sowie das über ELISA nachweisbare Digoxygenin-System, die über das Biotin/Avidin-System nachweisbare Biotin-Gruppe oder aber Linker-Arme mit funktionellen Gruppen, die eine nachträgliche Derivatisierung mit nachweisbaren Reporter-Gruppen gestatten, beispielsweise ein Aminoalkyl-Linker, der mit einem Acridinium-Aktivester zur Chemilumineszenz-Probe umgesetzt wird. Typische Markierungsgruppen sind:

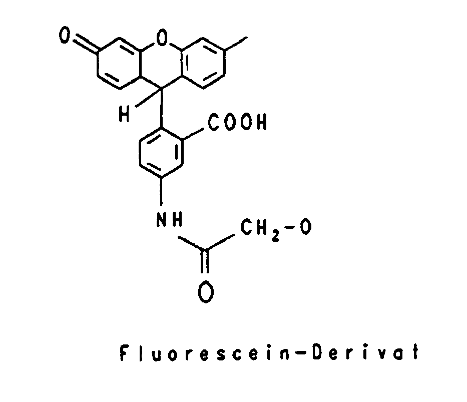

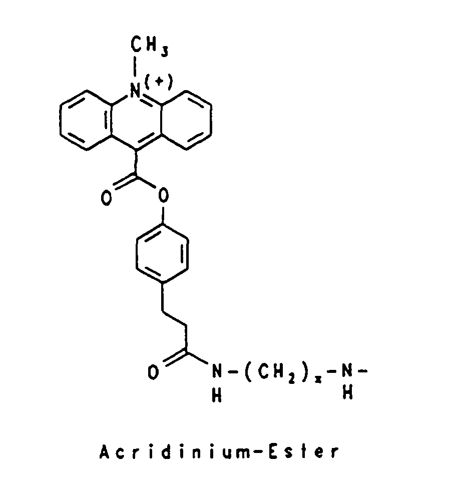

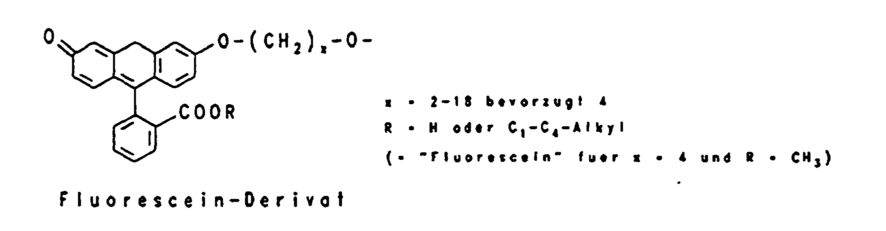

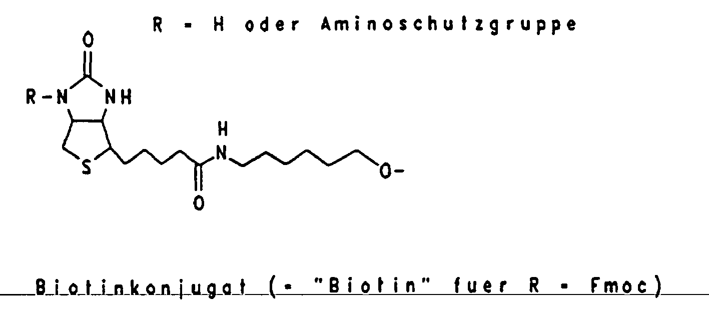

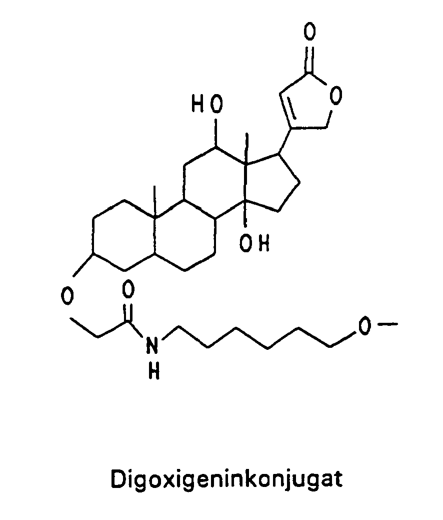

Oligonucleotidanaloga, die an Nucleinsäuren binden bzw. interkalieren und/oder spalten oder quervernetzen, enthalten z. B. Acridin-, Psoralen-, Phenanthridin, Naphtochinon-, Daunomycin- oder Chlorethylaminoaryl-Konjugate. Typische interkalierende und quervernetzende Reste sind:

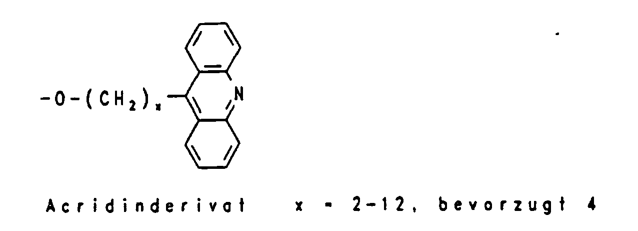

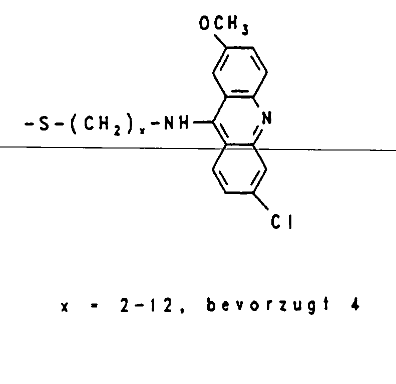

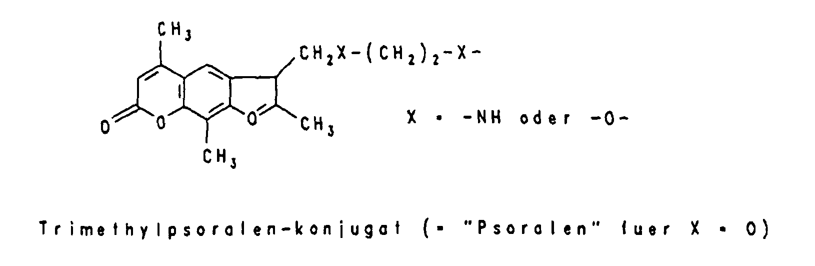

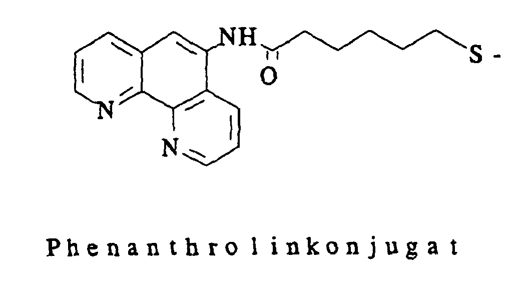

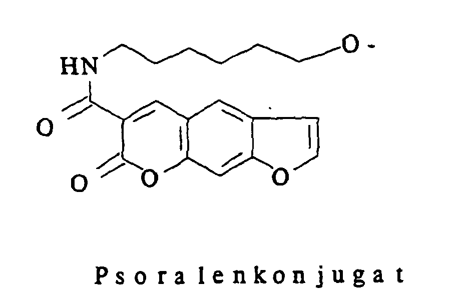

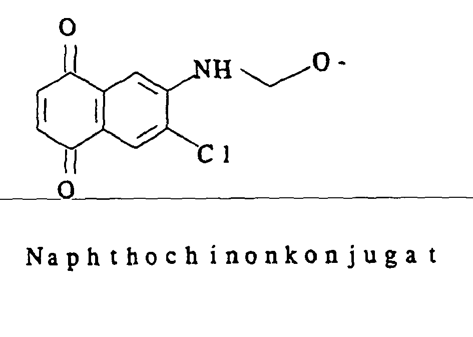

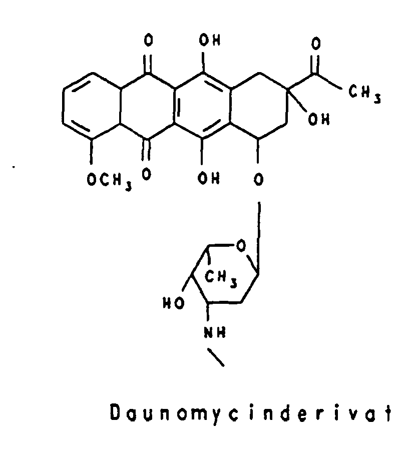

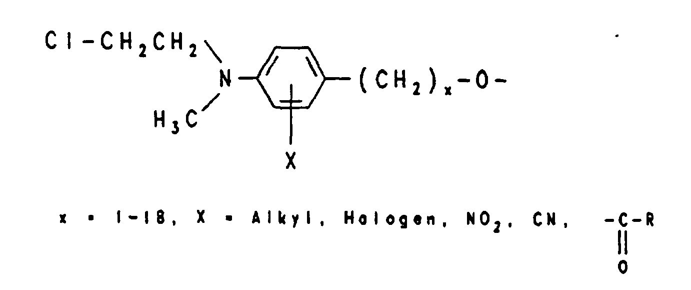

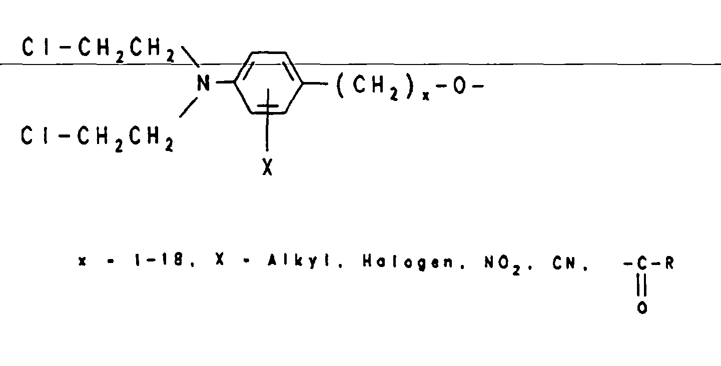

Beispiele für Gruppen NR³R⁴, in denen R³ und R⁴ zusammen mit dem sie tragenden Stickstoffatom einen 5- bis 6-gliedrigen heterocyclischen Ring bilden, der zusätzlich ein weiteres Heteroatom enthält, seien der Morpholinyl- und der Imidazolidinyl-Rest genannt.

Der Polyamid-Teil (PNA in Formel I) besteht aus Amid-Strukturen, welche mindestens eine Nucleobase enthalten, die von Thymin verschieden ist. Solche Polyamid-Strukturen sind beispielsweise aus folgenden Bausteinen a) bis h), bevorzugt a), aufgebaut, worin f für 1 bis 4, bevorzugt für 1 oder 2 und g für Null bis 3, bevorzugt für Null bis 2 stehen:
a) 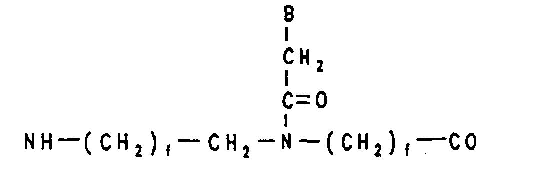 Hyrup et al. ; J. Chem. Soc. Chem. Comm. 1993, 519
b) 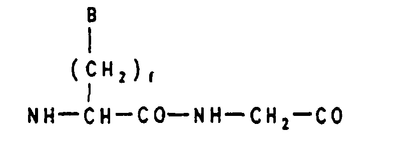 De Konig et al. (1971) Rec. Trav. Chim. 91, 1069
c) 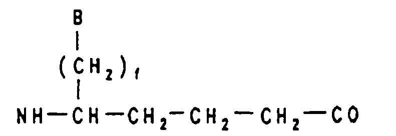 Huang et al. (1991) J. Org. Chem. 56, 6007
d) 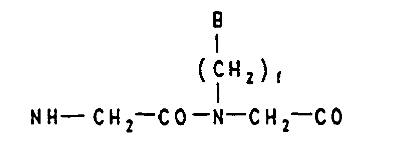 Almarsson et al. (1993) Proc. natl. Acad. Sci. U.S.A. 90, 7518
e) 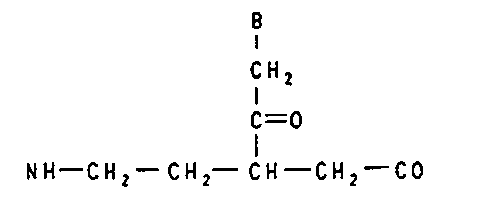 Froehler et al. (1991) WO 93/10820
f) 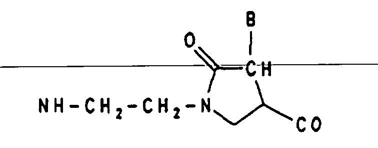 Froehler et al. (1991) WO 93/10820
g) 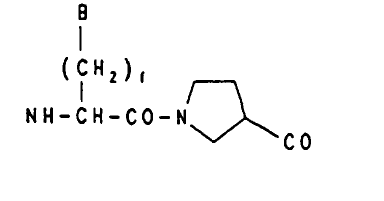 Lewis (1993) Tetrahedron Lett. 34, 5697.
h)

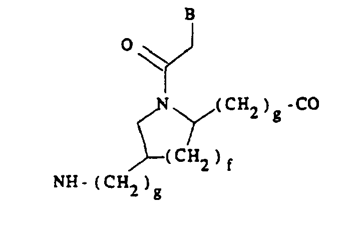

Endgruppen für PNA's sind in den gleichzeitig eingereichten Anmeldungen mit den Titeln "PNA-Synthese unter Verwendung einer gegen schwache Säuren labilen Amino-Schutzgruppe" (HOE 94/F 060, DE-P 44 08 531.1) und "PNA-Synthese unter Verwendung einer basenlabilen Amino-Schutzgruppe" (HOE 94/F 059, DE-P 44 08 533.8) beschrieben.

Bevorzugt sind Polyamidstrukturen, die aus Strukturen entsprechend a) aufgebaut sind. Besonders bevorzugt sind letztere, falls f=1 ist.

Die Darstellung von Polyamid-Oligonucleotid-Derivaten der Formel 1 erfolgt ähnlich wie die Synthese von Oligonucleotiden in Lösung oder vorzugsweise an fester Phase, gegebenenfalls unter Zuhilfenahme eines automatischen Synthesegeräts. Der Aufbau des Oligomers der Formel 1 kann schrittweise erfolgen, indem sukzessive eine PNA-Einheit bzw. DNA-Einheit mit jeweils einer Nucleobase an einen entsprechend derivatisierten Träger oder an eine wachsende Oligomerkette ankondensiert werden. Der Aufbau kann aber auch fragmentweise erfolgen, wobei die Fragmente zuerst als Polyamid- bzw. Oligonucleotid-Strukturen synthetisiert werden, welche dann zum Polyamid-Oligonucleotid der Formel I verknüpft werden. Es können jedoch auch Bausteine aus PNA und Nucleotid eingesetzt werden, vorzugsweise Dimere, die dann nach den Methoden der Nucleotid-Chemie oder Peptid-Chemie zu Polyamid-Oligonucleotid Derivaten aufgebaut werden.

Der Aufbau des Oligonucleotid-Teils erfolgt nach den dem Fachmann bekannten Verfahren wie der Triester-Methode, der H-Phosphonat-Methode oder Phosphoramidit-Methode, bevorzugt nach der Standard-Phosphoramidit Chemie nach Caruthers (M. D. Matteucci and M. H. Caruthers, J. Am. Chem. Soc. 103, 3185 (1981)). Die Synthese des Polyamid-Teils kann nach den dem Fachmann bekannten Methoden der Peptid-Chemie erfolgen. Falls Oligonucleotid-Teil und Polyamid-Teil nicht separat synthetisiert und nachfolgend verbunden werden, müssen die zum Aufbau der Oligonucleotid- und Polyamid-Struktur verwendeten Verfahren miteinader kompatibel sein, wobei eine bevorzugte-Ausführungsform zur Synthese des Polyamid-Teils in der gleichzeitig eingereichten Anmeldung mit dem Titel "PNA-Synthese unter Verwendung einer gegen schwache Säuren labilen Amino-Schutzgruppe (HOE 94/F 060, DE-P 44 08 531.1) beschrieben ist.

Je nachdem, ob q, r, s und t gleich 1 oder Null sind, erfolgt die Synthese beginnend mit dem Oligonucleotid- oder mit dem Polyamid-Teil. Die Synthese von Verbindungen der Formel l, deren Oligonucleotid-Teil am 3'- und/oder am 5'-Ende modifiziert sind, erfolgt bezüglich dieser Modifikationen nach den in EP-A 0 552 766 (HOE 92/F 012) beschriebenen Verfahren (vgl. Syntheseschema für DNA). Die Synthese von Verbindungen der Formel I erfolgt bezüglich des Polyamid-Teils nach dem in der gleichzeitig eingereichten Anmeldung mit dem Titel "PNA-Synthese unter Verwendung einer gegen schwache Säuren labilen Amino-Schutzgruppe (HOE 94/ F 060, DE-P 44 08 531.1) beschriebenen Verfahren (vgl. Syntheseschema für PNA).

Syntheseschema für DNA
[Ankergruppe]-[Polymer]
1. ↓ Aufkupplung von PG-(Nu')-aktiv
PG-(Nu')-[Ankergruppe]-[Polymer]
2. ↓ Abspaltung der Schutzgruppe PG
H-(Nu')-[Ankergruppe]-[Polymer]
3. ↓ Wiederholung der Schritte 1 und 2 (n-1)-mal
H-(Nu')n-[Ankergruppe]-[Polymer]
4. ↓ Aufkupplung von R0-V-aktiv
R0-V-(Nu')n-[Ankergruppe]-[Polymer]
5. ↓ Abspaltung von Polymer und Schutzgruppen
R0-V-(Nu)n

Syntheseschema für PNA

[Ankergruppe]-[Polymer]
1. ↓ Aufkupplung von PG-(Q')-OH
PG-(Q')-[Ankergruppe]-[Polymer]
2. ↓ Abspaltung der Schutzgruppe PG
H-(Q')-[Ankergruppe]-[Polymer]
3. ↓ Wiederholung der Schritte 1 und 2 (l-1)-mal
H-(Q')l-[Ankergruppe]-[Polymer]
4. ↓ Aufkupplung von PG-[B'/X]-OH
PG-[B'/X]-(Q')l-[Ankergruppe]-[Polymer]

5.	↓	Abspaltung der Schutzgruppe PG
H-[B'/X]-(Q')l-[Ankergruppe]-[Polymer]
6.	↓	Wiederholung der Schritte 4 und 5 (n-1)-mal
H-[B'/X]n-(Q')l-[Ankergruppe]-[Polymer]
7.	↓	Aufkupplung von PG-(A')-OH
PG-(A')-[B'/X]n-(Q')l-[Ankergruppe]-[Polymer]
8.	↓	Abspaltung der Schutzgruppe PG
H-(A')-[B'/X]n-(Q')l-[Ankergruppe]-[Polymer]
9.	↓	Wiederholung der Schritte 7 und 8 (k-1)-mal
H-(A')k-[B'/X]n-(Q')l-[Ankergruppe]-[Polymer]
10.	↓	Aufkupplung der Gruppe R0
R0-(A')k-[B'/X]n-(Q')l-[Ankergruppe]-[Polymer]
11.	↓	Abspaltung von Polymer und Schutzgruppen
R0-(A)k-[B/X]n-(Q)l-Q°

### Darin bedeuten:

PG Schutzgruppe, bevorzugt eine gegen schwache Säure labile Schutzgruppe;
Nu' Nucleotid-Einheit, deren exocyclische Aminogruppe durch eine geeignete Schutzgruppe geschützt ist;
Nu'-aktiv ein in der Nucleotidchemie übliches aktiviertes Derivat, wie z.B. von einem Phosphoramidit, einem Phosphordiester oder einem H-Phosphonat; R', B' und Q' stehen für die gegebenenfalls geschützten Formen von A, B und Q.

### Syntheseschema für PNA/DNA-Hybride der Formel I

F[(DNA-Li)_{q}(PNA-Li)ᵣ(DNA-Li)ₛ(PNA)ₜ]ₓF' (I)

Für q=r=s=t=1 und x=1 gilt folgender Syntheseablauf:

1. ↓ Synthese der Endgruppe F'; ggf. Konjugation auf Polymer
PG-F'
2. ↓ Abspaltung der Schutzgruppe PG
H-F'
3. ↓ Konjugation der Polyamidstruktur
PNA-F'
4. ↓ Aufkupplung eines Linkers
Li-PNA-F'
5. ↓ Konjugation der Nucleotidstruktur
DNA-Li-PNA-F'
6. ↓ Aufkupplung eines Linkers
Li-DNA-Li-PNA-F'
7. ↓ Wiederholung der Schritte 3 bis 5
DNA-Li-PNA-Li-DNA-Li-PNA-F'
8. ↓ Aufkupplung der Endgruppe F
F-DNA-Li-PNA-Li-DNA-Li-PNA-F'

Die Aufkupplung des Linkerbausteins kann entfallen, sofern sich entsprechende Übergänge in den PNA- oder DNA-Bausteinen befinden.

Zur Verdeutlichung ist ein Syntheseschema für PNA/DNA-Hybride der Formel I gezeigt, das die Herstellung eines Hybrid-Oligomers, in dem q=r=s=t=1 und x=1 sind, beispielhaft erläutert. Zunächst wird die Endgruppe F nach bekannten Verfahren synthetisiert und im Falle der Festphasen-Synthese auf einen polymeren Träger gekuppelt (Schritt 1). Nach Abspaltung der Schutzgruppe PG (Schritt 2), die bevorzugt im schwach sauren Medium erfolgt, werden die Polyamid-Bausteine bis zur gewünschten Länge des PNA-Teils aufgekuppelt (Schritt 3) . Als Übergang zum DNA-Teil kann nun die Anknüpfung einer Linker-Einheit (Schritt 4) erfolgen. Anschließend erfolgt die Konjugation der Nucleotidstruktur durch sukkzessive Ankondensation der Nucleotid-Bausteine (Schritt 5), bevorzugt nach der bekannten Phosphoramidit-Methode. Nach Ankondensation eines Linkers (Schritt 6), der den Übergang von DNA zu PNA ermöglicht, wird wiederum eine Polyamidstruktur aufgebaut. Einführung eines Linkers, der den Übergang von PNA nach DNA ermöglicht, Konjugation einer weiteren DNA-Struktur (Schritt 7) und abschließende Aufkupplung der Endgruppe F (Schritt 8) ergeben das Hybridmolekül [F-DNA-Li-PNA-Li-DNA-Li-PNA-F']. Die Linkerbausteine können hierbei auch Nucleobasen beinhalten. Zur Synthese eines Hybrids F-DNA-Li-PNA-Li-F' (q=r=1, s=t=Null) werden beispielsweise erst die Schritte 1-5 durchgeführt und die Synthese dann mit Schritt 8 abgeschlossen.
Zur Synthese eines Hybrids F-PNA-Li-DNA-F' (r=s=1, q=t=Null) werden beispielsweise erst die Schritte 1-2 durchgeführt, danach folgen die Schritte 5-6, gefolgt von Schritt 3 und Abschluß der Synthese mit Schritt 8. Zur Synthese eines Hybrids F-PNA-Li-DNA-Li-PNA-F' (r=s=t=1, q = Null) beginnt die Synthese mit den Schritten 1-6. Nach Wiederholung des Schrittes 3 wird die Synthese mit Schritt 8 abgeschlossen.
Ist x in Formel I > 1, dann müssen die Schritte 2-7 gegebenfalls wiederholt werden. Nach Aufbau der polymeren ketten müssen die PNA/DNA-Hybride im Falle der Festphasensynthese vom Träger abgespalten werden und gegenenfalls die Schutzgruppen an den Basen, Aminosäure-Seitenketten und Endgruppen abgespalten werden.
PNA- und DNA-Teil können aber auch getrennt nach bekannten Methoden synthetisiert und anschließend über entsprechende Aktivierung mindestens einer Komponente miteinander gekuppelt werden. Die Aktivierung des PNA-Teils erfolgt bevorzugt über die Carbonsäure-Gruppe, beispielsweise als Aktivester oder Isothiocyanat, die dann mit reaktiven Gruppen im DNA-Teil, vorzugsweise einer Aminofunktion in Reaktion gebracht werden. Die Aktivierung des DNA-Teils erfolgt beispielsweise in Form einer an sich bekannten Bromcyan-Kondensation, bei der die aktivierte Phosphatfunktion mit einer reaktiven Gruppe im PNA-Teil, bevorzugt einer Aminofunktion zur Reaktion gebracht wird.

Überraschenderweise wurde gefunden, daß die Oligomeren der Formel la und lb im Vergleich zu den reinen PNA's eine stark erhöhte zelluläre Aufnahme besitzen. Diese verbesserte Zellaufnahme ist ganz entscheidend, da Antisense- oder Triplex-bildende Oligomere nur dann wirken können, wenn sie effektiv in Zellen aufgenommen werden. Ihr Hybridisationsverhalten ist ebenfalls günstiger als bei reinen PNA's, da sie bevorzugt zur antiparallelen Duplexbildung führen. Im Vergleich zu normalen Oligonucleotiden besitzen sie eine verbesserte Nucleasestabilität, was sich in einer erhöhten biologischen Aktivität äußert. Die Bindungsaffinität an komplementäre Nucleinsäuren ist besser als die anderer nucleasestabiler Oligonucleotide, wie z.B. Phosphorothioate oder Methylphosphonate. Die Bindungsaffinität der erfindungsgemäßen Verbindungen ist beim Vergleich mit natürlichen Oligonucleotiden, die unter Serum-Bedingungen rasch abgebaut werden, mindestens gleich gut oder meistens jedoch besser. Die Erhöhung der Bindungsaffinität ist abhängig von der Länge des PNA-Teils. Reine PNA's zeigten in den Zellkulturversuchen bei Konzentrationen von > 5 *µ*M stark cytotoxische Wirkung, während die erfindungsgemäßen Verbindungen die Zellen nicht schädigten. Es wurde ferner gefunden, daß Verbindungen der Formel I in Abhangigkeit von der üasenfotge des PNA- und DNA-Teils die Expression spezifischer Gene, beispielsweise von Enzymen, Rezeptoren oder Wachstumsfaktoren in Zellkultur und in ausgewählten Beispielen im Tiermodell hemmen.

Weitere Vorteile der PNA/DNA-Oligomeren bzw. PNA/RNA-Oligomeren bestehen in der Möglichkeit der Stimmulierung zellulärer Endonucleasen wie beispielsweise RNase H und RNase L. Im Gegensatz zu PNA's können die erfindungsgemäßen PNA-DNA-Chimären, die einige Desoxyribonucleotid-Einheiten aufweisen, nach Anbindung an die komplementäre Target-RNA diese in sequenzspezifischer Weise durch Induktion der zellulären RNase H spalten. Eine besondere Ausführungsform der erfindungsgemäßen Oligomeren sind weiterhin solche, die aus PNA- und einem 2'5'-verknüpften Oligoadenylat-Teil, vorzugsweise Tetraadenylat oder dessen Cordycepin-Analogon aufgebaut sind, und die die zelluläre RNase L aktivieren.

Ganz generell erstreckt sich die vorliegende Erfindung auf die Verwendung von Verbindungen der Formel 1 als therapeutisch wirksame Bestandteile eines Arzneimittels. Als therapeutisch wirksame Polyamid-Oligonucleotid-Derivate versteht man im allgemeinen Antisense Oligonucleotide, Tripelhelix-bildende-Oligonucleotide, Aptamere oder Ribozyme, insbesondere Antisense-Oligonucleotide.

Die Arzneimittel der vorliegenden Erfindung können beispielsweise zur Behandlung von Erkrankungen, die durch Viren hervorgerufen werden, beispielsweise durch HIV, HSV-1, HSV-2, Influenza, VSV, Hepatitis B oder Papilloma Viren, verwendet werden.

Erfindungsgemäße Antisense Polyamid-Oligonucleotide-Derivate, die gegen solche Targets wirksam sind, haben beispielsweise folgende Basensequenz. Die Länge und Position des PNA-und DNA-Teils kann in diesen Sequenzen zur Erzielung optimaler Eigenschaften entsprechend variiert werden.
a) gegen HIV, z. B.
   5'-ACACCCAATTCTGAAAATGG-3' oder (I)
   5'-AGGTCCCTGTTCGGGCGCCA-3' oder (II)
   5'-GTCGACACCCAATTCTGAAAATGGATAA-3' oder (III)
   5'-GCTATGTCGACACCCAATTCTGAAA-3' oder (IV)
   5'-TCGTCGCTGTCTCCGCTTCTTCTTCCTGCCA oder (VI)
b) gegen HSV-1, z.B.
   5'-GCGGGGCTCCATGGGGGTCG-3' (VII)

Die Arzneimittel der vorliegenden Erfindung eignen sich beispielsweise auch zur Behandlung von Krebs. Beispielsweise können dabei Polyamid-Oligonucleotid-Sequenzen zum Einsatz kommen, die gegen Targets gerichtet sind, die für Krebsentstehung bzw. Krebswachstum verantwortlich sind. Solche Targets sind beispielsweise:
1) Nucleare Onkoproteine wie beispielsweise c-myc, N-myc, c-myb, c-fos, c-fos/jun, PCNA, p120
2) Cytoplasmische/Membran-assoziierte Onkoproteine wie beispielsweise EJras, c-Ha-ras, N-ras, rrg, bcl-2, cdc-2, c-raf-1, c-mos, c-src, c-abl
3) Zelluläre Rezeptoren wie beispielsweise EGF-Rezeptor, c-erbA, Retinoid-Rezeptoren, Protein-Kinase regulatorische Untereinheit, c-fms
4) Cytokine, Wachstumsfaktoren, Extrazelluläre Matrix wie beispielsweise CSF-1, IL-6, IL-1a, IL-1b, IL-2, IL-4, bFGF, Myeloblastin, Fibronectin,

Erfindungsgemäße Antisense-Polyamid-Oligonucleotide der Formel 1, die gegen solche Targets wirksam sind, haben beispielsweise folgende Basen-Sequenz:
a) gegen c-Ha-ras, z. B.
   6'-CAGCTGCAACCCAGC-3'
   (VIII)
b) bFGF, z.B.
   5'-GGCTGCCATGGTCCC-3'
   (XXX)
c) c-myc, z.B.
   5'-GGCTGCTGGAGCGGGGCACAC-3'
   (IX)
   5'-AACGTTGAGGGGCAT-3'
   (X)
d) c-myb, z.B.
   5'-GTGCCGGGGTCTTCGGGC-3'
   (XI)
e) c-fos, z.B.
   5'-GGAGAACATCATGGTCGAAAG-3'
   (XII)
   5'-CCCGAGAACATCATGGTCGAAG-3'
   (XIII)
   5'-GGGGAAAGCCCGGCAAGGGG-3'
   (XIV)
f) p 120, z.B.
   5'-CACCCGCCTTGGCCTCCCAC-3'
   (XV)
g) EGF-Rezeptor, z.B.
   5'-GGGACTCCGGCGCAGCGC-3'
   (XVI)
   5'-GGCAAACTTTCTTTTCCTCC-3'
   (XVII)
h) p53 Tumorsuppressor, z.B.
   5'-GGGAAGGAGGAGGATGAGG-3'
   (XVIII)
   5'-GGCAGTCATCCAGCTTCGGAG-3'r
   (XIX)

Die Arzneimittel der vorliegenden Erfindung eignen sich beispielsweise ferner zur Behandlung von Erkrankungen, die durch Integrine oder Zell-Zell-Adhäsionsrezeptoren beeinflußt werden, beispielsweise durch VLA-4, VLA-2, ICAM, VCAM oder ELAM.

Erfindungsgemäße Antisense-Polyamid-Oligonucleotid-Derivate, die gegen solche Targets wirksam sind, haben beispielsweise folgende Basen-Sequenz:
a) VLA-4, z.B.
   5'-GCAGTAAGCATCCATATC-3' oder
   (XX)
b) ICAM, z.B.
   5'-CCCCCACCACTTCCCCTCTC-3'
   (XXI)
   5'-CTCCCCCACCACTTCCCCTC-3'
   (XXII)
   5'-GCTGGGAGCCATAGCGAGG-3'
   (XXIII)
c) ELAM-1, z. B.
   5'-ACTGCTGCCTCTTGTCTCAGG-3'
   (XXIV)
   5'-CAATCAATGACTTCAAGAGTTC-3'
   (XXV)

Die Arzneimittel der vorliegenden Erfindung eignen sich beispielsweise auch zur Verhinderung der Restenose. Beispielsweise können dabei Polyamid-Oligonucleotid-Sequenzen
zum Einsatz kommen, die gegen Targets gerichtet sind, die für Proliferation oder Migration verantwortlich sind. Solche Targets sind beispielsweise:
1) Nucleare Transaktivator-Proteine und Cycline wie beispielsweise c-myc, c-myb, c-fos, c-fos/jun, Cycline und cdc2-Kinase
2) Mitogene oder Wachstumsfaktoren wie beispielsweise PDGF, bFGF, EGF, HB-EGF und TGF-β.
3) Zelluläre Rezeptoren wie beispielsweise bFGF-Rezeptor, EGF-Rezeptor und PDGF-Rezeptor.

Erfindungsgemäße Antisense-Polyamid-Oligonucleotide der Formel 1, die gegen solche Targets wirksam sind, haben beispielsweise folgende Basen-Sequenz:
a) c-myb
   5'-GTGTCGGGGTCTCCGGGC-3'
   (XXVI)
b) c-myc
   5'-CACGTTGAGGGGCAT-3'
   (XXVII)
c) cdc2-Kinase
   5'-GTCTTCCATAGTTACTCA-3'
   (XXVIII)
d) PCNA (proliferating cell nuclear antigen of rat)
   5'-GATCAGGCGTGCCTCAAA-3'
   (XXIX)

Die Arzneimittel können z.B. in Form von pharmazeutischen Präparaten, die man oral, z.B. in Form von Tabletten, Dragees, Hart- oder Weichgelatinekapseln, Lösungen, Emulsionen oder Suspensionen verabreichen kann, verwendet werden. Der Einschluß der Arzneimittel in Liposomen, die gegebenenfalls weitere Komponenten wie Proteine enthalten, ist eine ebenfalls geeignete Applikationsform. Sie können auch rektal z.B. in Form von Suppositorien oder parenteral z.B. in Form von Injektionslösungen verabreicht werden. Für die Herstellung von pharmazeutischen Präparaten können diese Verbindungen in therapeutisch inerten organischen und anorganischen Trägern verarbeitet werden. Beispiele von solchen Trägern für Tabletten, Dragees und Hartgelatinekapseln sind Laktose, Maisstärke oder Derivate davon, Talk und Stearinsäure oder Salze davon. Geeignete Träger für die Herstellung von Lösungen sind Wasser, Polyole, Saccharose, Invertzucker und Glucose. Geeignete Träger für Injektionslösungen sind Wasser, Alkohole, Polyole, Glycerol und pflanzliche Öle. Geeignete Träger für Suppositorien sind pflanzliche und gehärtete Öle, Wachse, Fette und halbflüssige Polyole. Die pharmazeutischen Präparate können auch Konservierungsmittel, Lösemittel, Stabilisierungsmittel, Netzmittel, Emulgatoren, Süßstoffe, Farbstoffe, Geschmacksmittel, Salze zur Veränderung des osmotischen Drucks, Puffer, Überzugsmittel, Antioxidantien, sowie ggf. andere therapeutische Wirkstoffe enthalten.

Bevorzugte Verabreichungsformen sind topische Applikationen, lokale Applikationen wie beispielsweise mit Hilfe eines Katheters oder aber auch Injektionen. Zur Injektion werden die Antisense-Polyamid-Oligonucleotid-Derivate in einer flüssigen Lösung, vorzugsweise in einem physiologisch annehmbaren Puffer, wie z.B. Hank's Lösung oder Ringer's Lösung, formuliert. Die Antisense-Polyamid-Oligonucleotide können aber auch in fester Form formuliert werden und vor dem Gebrauch gelöst oder suspendiert werden. Die für die systemische Verabreichung bevorzugten Dosierungen betragen ca. 0,01 mg/kg bis ca. 50 mg/kg Körpergewicht und Tag.

Ganz generell erstreckt sich die Erfindung auf die Verwendung von Verbindungen der Formel I als DNA-Sonden oder Primer in der DNA-Diagnostik, insbesondere im Sinne der in HOE 92/F 406 (EP-A 0 602 524) genannten Gensonden, und allgemein als Hilfsmittel in der Molekularbiologie.

In der DNA-Diagnostik spielen Gensonden, auch DNA-Probes oder Hybridization-Probes genannt, eine große Rolle zum sequenzspezifischen Nachweis bestimmter Gene. Eine Gensonde besteht im allgemeinen aus einer Erkennungssequenz und einer geeigneten Markierungsgruppe (Label). Die Spezifität der Bestimmung einer Targetsequenz in einer Analysenprobe mittels Hybridisierung mit einer komplementären Gensonde wird durch die Erkennungssequenz und deren chemischer Struktur determiniert. Die PNA's haben gegenüber den Oligonucleotiden natürlicher Struktur den Vorteil, daß sie eine höhere Affinität zur Targetsequenz besitzen. Jedoch ist die Spezifität der Hybridisierung reduziert, da PNA's im Gegensatz zu natürlicher DNA sowohl in paralleler als auch in antiparalleler Orientierung an einzelsträngige Nucleinsäuren binden können. Die erfindungsgemäßen PNA/DNA-Oligomeren zeigen ebenfalls eine erhöhte Bindungsaffinität, binden jedoch stark bevorzugt in der gewünschten antiparallelen Orientierung.

Durch entsprechende Auswahl des PNA- bzw. DNA-Teils in einer Gensonde kann zudem das Differenzierungsvermögen positiv beeinflußt werden, da eine Basenmißpaarung im PNA-Teil zu einer stärkeren Depression der Schmelztemperatur eines Hybrids führt als eine Basenmißpaarung im DNA-Teil. Dies ist besonders wichtig im Hinblick auf die Differenzierung bei Punktmutationen wie sie beispielsweise beim Übergang von Protoonkogenen in die entsprechenden Onkogene (pathogener Zustand) vorkommen. Der Vorteil der besseren Diskriminierung zwischen pathogenem und nichtpathogenem Zustand läßt sich auch in Form der Primer-Eigenschaft der erfindungsgemäßen PNA/DNA-Oligomeren nutzen, sofern diese eine freie 3'-Hydroxyfunktion im DNA-Teil besitzen. PNA's als solche besitzen keine Primer-Funktion für Polymerasen. Überrasschenderweise wurde gefunden, daß bereits eine Nucleosid-Einheit am Ende eines PNA/DNA-Oligomers ausreicht, um die DNA-Polymerasereaktion, beispielsweise mit Hilfe der DNA-Polymerase (Klenow-Fragment) zu initiieren. Je nach Beschaffenheit des PNA/DNA-Primers und der Art des Templats, an welches der Primer in sequenzspezifischer Weise hybridisiert, können verschiedene Polymerasen eingesetzt werden. Diese sind im allgemeinen komerziell zugänglich, wie beispielsweise Taq-Polymerase, Klenow-Polymerase oder Reverse Transkriptase.

Ein weiterer Vorteil gegenüber der Verwendung natürlicher Oligonucleotid-Primer besteht darin, daß der mit Hilfe des PNA/DNA-Primers kopierte Nucleinsäurestrang, der den PNA-Teil am 5'-Ende enthält, gegen 5'-Exonucleasen stabil ist. Somit können alle natürlichen DNA- bzw. RNA-Sequenzen im Reaktionsgemisch durch 5'Exonucleasen abgebaut werden, ohne daß der PNA-haltige Strang angegriffen wird.

Ein weiterer Vorteil der PNA/DNA-Oligomeren besteht darin, daß man damit auch andere biochemische Reaktionen am DNA-Teil durchführen kann, welche bei PNA's selbst nicht möglich sind. Beispiele für solche Reaktionen sind das "3'-Tailing" mit 3'-terminaler Transferase, der Restriktionsenzymverdau im DNA-Doppelstrang-Bereich sowie Ligase-Reaktionen. Beispielsweise kann ein (PNA)-(DNA)-OH Oligomer mit freier 3'-Hydroxygruppe mit einem zweiten p-(DNA)-(PNA)-Oligomer, das am 5'-Ende ein Nucleosid-5'-Phosphat enthält, nach Hybridisierung an eine komplementäre DNA-Hilfssequenz natürlichen Ursprungs in Gegenwart einer DNA-Ligase verknüpft werden.

Weiterhin lassen sich (DNA)-(PNA)-(DNA) Oligomere in Gene einbauen, was mit PNA's zur Zeit nicht möglich ist.

Die Anknüpfung von Markierungsgruppen an PNA/DNA-Oligomere erfolgt nach an sich bekannten Methoden, wie sie für Oligonucleotide oder Peptide beschrieben sind. Die Art der Markierungsgruppe kann breit variiert werden und richtet sich im wesentlichen nach der Art des verwendeten Assays. Bekannte Ausführungsformen von Gensonden-Assays sind "Hybridization Protection"-, "Energy-Transfer"- und "Kissing Probes"-Assay. PNA/DNA-Oligomere eignen sich zudem besonders gut für einen "Strand Displacement"-Assay. In vielen Fällen ist die Abtrennung des gebildeten Hybrids von überschüssiger Gensonde mit Hilfe von Magnetpartikeln von Vorteil. Die Stabilität der erfindungsgemäßen PNA/DNA-Gensonden ist höher als die herkömmlicher DNA-Sonden.

"Polymerase Chain Reaction" (PCR) und "Ligase Chain Reaction" (LCR) sind Techniken zur Targetamplifikation, in welche die erfindungsgemäßen Oligomeren ebenfalls als Primer eingesetzt werden können. Besonders vorteilhaft lassen sich die PNA/DNA-Oligomeren als Gensonden nach dem "Christmas tree"-Prinzip verwenden, da hierbei die PNA/DNA-Sonden kürzer sein können als entsprechende DNA-Sonden.

### Beispiele:

Die für Aminosäuren verwendeten Abkürzungen entsprechen dem in der Peptidchemie üblichen drei Buchstaben-Code wie er in Europ. J. Biochem. 138, 9 (1984) beschrieben ist. Weitere verwendete Abkürzungen sind nachfolgend aufgelistet.
- Aeg: N-(2-Aminoethyl)glycyl, -NH-CH₂-CH₂-NH-CH₂-CO-
- Aeg (a^{MeOBz}): N-(2-Aminoethyl)-N-((9-(N⁶-4-methoxybenzoyl)-adenosyl)acetyl)-glycyl
- Aeg (c^{Bz}): N-(2-Aminoethyl)-N-((1-(N⁴-benzoyl)-cytosyl)acetyl)-glycyl
- Aeg(c^{MeOBz}): N-(2-Aminoethyl)-N-((1-(N⁴-4-methoxybenzoyl)-cytosyl)acetyl)-glycyl
- Aeg(c^{tBuBz}): N-(2-Aminoethyl)-N-((1-(N⁴-4-tert.butylbenzoyl)-cytosyl)acetyl)-glycyl
- Aeg(g^{iBu}): N-(2-Aminoethyl)-N-((9-(N²-isobutanoyl)-guanosyl)acetyl)-glycyl
- Aeg (g^{2-Ac,4-Dpc}): N-(2-Aminoethyl)-N-((9-(N²-acetyl-O⁴⁻diphenylcarbamoyl)guanosyl)glycyl
- Aeg (t): N-(2-Aminoethyl)-N-((1-thyminyl)acetyl)-glycyl
- Bnpeoc: 2,2-[Bis(4-nitrophenyl)]-ethoxycarbonyl)
- Boc: tert.-Butyloxycarbonyl
- BOI: 2-(Benzotriazol-1-yl)oxy-1,3-dimethyl-imidazolidinium-hexafluorphosphat
- BOP: Benzotriazolyl-1-oxy-tris(dimethylamino)-phosphonium-hexafluorphosphat
- BroP: Brom-tris(dimethylamino)-phosphonium-hexafluorphosphat
- BSA: N,O-Bis-(trimethylsilyl)-acetamid
- But: tert.-Butyl
- Bz: Benzoyl
- Bzl: Benzyl
- CI-Z: 4-Chlor-benzyloxycarbonyl
- CPG: Controlled Pore Glas
- DBU: 1, 8-Diazabicyclo[5.4.0]undec-7-en
- DCM: Dichlormethan
- Ddz: 3.5-Dimethoxyphenyl-2-propyl-2-oxycarbonyl
- DMF: Dimethylformamid
- Dmt: Di-(4-methoxyphenyl)phenylmethyl,
- Dnpeoc: 2-(2,4-Dinitrophenyl)-ethoxycarbonyl
- Dpc: Diphenylcarbamoyl
- FAM: Fluorescein-Rest
- Fm: 9-Fluorenylmethyl
- Fmoc: 9-Fluorenylmethyloxycarbonyl
- H-Aeg-OH: N-(2-Aminoethyl)glycin
- HAPyU: O-(7-Azabenzotriazol-1-yl)-1,1,3,3-bis(tetramethylen)uronium-hexafluorphosphat
- HATU: O-(7-Azabenzotriazol-1-yl)-1,1,3,3-tetramethyluronium-hexafluorphosphat
- HBTU: O-(Benzotriazol-1-yl)-1,1,3,3-tetramethyluronium-hexafluorphosphat
- HOBt: 1-Hydroxybenzotriazol
- HONSu: N-Hydroxysuccinimid
- HOObt: 3-Hydroxy-4-oxo-3,4- dihydrobenzotriazin
- iBu: Isobutanoyl
- MeOBz: 4-Methoxybenzoyl
- Mmt: 4-Methoxytriphenylmethyl
- Moz: 4-Methoxybenzyloxycarbonyl
- MSNT: 2,4,6-Mesitylensulfonyl-3-nitro-1,2,4-triazolid
- Mtt: 4-Methylphenyl)diphenylmethyl
- NBA: Nitrobenzylalkohol
- NMP: N-Methylpyrrolidin
- Obg: N-(4-Oxybutyl)glycyl, -O-(CH₂)₄-NH-CH₂-CO-
- Obg(t): N-(4-Oxybutyl)-N-((1-thyminyl)acetyl)-glycyl
- Oeg: N-(2-Oxyethyl)glycyl, -O-CH₂-CH₂-NH-CH₂-CO-
- Oeg(t): N-(2-Oxyethyl)-N-((1-thyminyl) acetyl)-glycyl
- Opeg: N-(5-Oxypentyl)glycyl, -O-(CH₂)₅-NH-CH₂-CO-
- Opeg(t): N-(5-Oxypentyl)-N-((1-thyminyl) acetyl)-glycyl
- Oprg: N-(3-Oxypropyl)glycyl, -O-(CH₂)₃-NH-CH₂-CO-
- Oprg(t): N-(3-Oxypropyl)-N-((1-thyminyl)acetyl)-glycyl
- Pixyl: 9-(9-Phenyl)xanthenyl
- PyBOP: Benzotriazolyl-1-oxy-tripyrrolidinophosphonium-hexafluorphosphat
- PyBroP: Brom-tripyrrolidinophosphonium-hexafluorphosphat
- TAPipU: O-(7-Azabenzotriazo)-1-y)-1,1,3,3-bis(pentamethylen)uronium-tetrafluoroborat
- TBTU: O-(Benzotriazol-1-yl)-1,1,3,3-tetramethyluronium-tetrafluoroborat
- tBu: tert.-Butyl
- tBuBz: 4-tert.Butylbenzoyl
- TDBTU: O-(3,4-Dihydro-4-oxo-1,2,3-benzotriazin-3-yl)-1,1,3,3-tetramethyluronium-tetrafluoroborat
- TDO: 2,5-Diphenyl-2,3-dihydro-3-oxo-4-hydroxythiophendioxid
- Teg: N-(2-Thioethyl)glycyl, -S-CH₂-CH₂-NH-CH₂-CO-
- Teg(t): N-(2-Thioethyl)-N-((1-thyminyl)acetyl)-glycyl
- TFA: Trifluoressigsäure
- THF: Tetrahydrofuran
- TNTU: O-[(5-Norbornen-2,3-dicarboximido]-1,1,3,3-tetramethyluronium-tetrafluoroborat
- TOTU: O-[(Cyano(ethoxycarbonyl)methylen)amino]-1,1,3,3-tetramethyluronium-tetrafluoroborat
- TPTU: O-(1,2-Dihydro-2-oxo-1-pyridyl)-1,1,3,3'-tetramethyluronium-tetrafluoroborat
- Trt: Trityl
- TSTU: O-(N-Succinimidyl)-1,1,3,3-tetramethyluronium-tetrafluoroborat
- Z: Benzyloxycarbonyl
- MS(ES⁺): Elektrospray Massenspektrum (Positiv Ion)
- MS(ES⁻): Elektrospray Massenspektrum (Negativ Ion)
- MS(DCI): Desorption Chemical lonisation Massenspektrum
- MS(FAB): East-Atom-fiombardment-Massenspektrum

### Beispiel 1

### 1-Hydroxy-6-((4-methoxyphenyl)-diphenylmethylamino)-hexan Mmt-hex

6-Aminohexan-1-ol (1g; 8.55mmol) wird in wasserfreiem Pyridin (7ml) gelöst und mit Triethylamin (0.2ml) versetzt. Zu dieser Lösung gibt man innerhalb von 45 Minuten eine Lösung von (4-Methoxyphenyl)-diphenylmethyl chlorid (2.5g; 8.12mmol) in wasserfreiem Pyridin (9ml). Die Reaktionslösung wird 30 Minuten bei 22°C weitergerührt und durch Zugabe von Methanol (3ml) gestoppt. Die Lösung wird am Rotationsverdampfer eingeengt, der erhaltene Rückstand zur Entfernung des Pyridins dreimal mit Toluol koevaporiert. Der erhaltene Rückstand wird in Ethylacetat gelöst und diese Lösung nacheinander mit einer gesättigten Natriumbicarbonatlösung, mit Wasser und einer gesättigten Kaliumchloridlösung gewaschen. Die organische Phase wird getrocknet (Na₂SO₄), filtriert und im Vakuum konzentriert. Das Rohprodukt wird durch Kieselgelchromatographie mit Heptan:Ethylacetat:Triethylamin/49.5:49.5:1 gereinigt.
Ausbeute: 1.64g
MS (FAB,NBA/LiCl) 396.3 (M+Li)⁺, 390.3 (M+H)⁺, 273.2 (Mmt)⁺
R_{f} 0.44 (Heptan:Ethylacetat =1:1),

### Beispiel 2

### 6-((4-Methoxyphenyl)-diphenylmethylamino)-hex-1-yl hemisuccinat Mmt-hex-succ

1-Hydroxy-6-((4-methoxyphenyl)-diphenylmethylamino)-hexan (1.00g; 2.57mmol) wird in wasserfreiem Pyridin (10ml) gelöst. Zu dieser Lösung gibt man Bernsteinsäureanhydrid (0.257g; 2.57mmol) and 4-Dimethylaminopyridin (31.3mg; 0.257mmol). Nach 3 Stunden Rühren bei 22°C gibt man weiteres Bernsteinsäureanhydrid (25.7mg; 0.257mmol) und 4-Dimethylaminopyridin (62.6mg; 0.56mmol) zu und erwärmt diese Lösung 6 Stunden lang auf 50°C. Nach weiteren 16 Stunden bei 22°C wird eingeengt, der Rücksatnd in Ethylacetat aufgenommen und die erhaltene Lösung mit eiskalter 5%-iger wässriger Zitronensäure gewaschen. Nach Trocknen der org. Phase (Na₂SO₄) wird die Lösung am Rotationsverdampfer eingeengt. Die Reinigung des Rückstands durch Kieselgelchromatographie mit 50% CH₂Cl₂/1% Triethylamin in Ethylacetat und dann mit 5% Methanol/1% Triethylamin in Dichloromethan ergibt die gewünschte Verbindung als farbloses Öl.
MS (ES⁻) 978.0 (2M-H)⁻, 488.3 (M-H)⁻
R_{f} 0.30 (CH₂Cl₂: EthylAcetat = 1:1).

### Beispiel 3

### 6-((4-Methoxyphenyl)-diphenylmethylamino)-hex-1-yl succinylamido-Tentagel (Mmt-hex-succ-Tentagel)

Die Aminoform von Tentagel^{R} (Rapp Polymere) (0.5g; 0.11mmol Aminogruppen) läßt man 10 Minuten in 4-Ethylmorpholin (0.1ml) und DMF (5ml) quellen. Dann gibt man eine Lösung von 6-((4-Methoxyphenyl)-diphenylmethylamino)-hex-1-yl hemisuccinat (97.4mg; 0.165mmol), 4-Ethylmorpholin (15.9mg; 0.138mmol; 17.4ml) and TBTU (52.9mg; 0.165mmol) in DMF (3ml) zu und schüttelt die Suspension 16 Stunden lang bei 22°C. Der derivatisierte Tentagel-Träger wird abfiltriert und nacheinander mit DMF (3x3ml), CH₂Cl₂ (3x1ml) and Diethylether (3x1 ml) gewaschen und getrocknet. Nicht reagierte Aminofunktionen werden durch 1-stündige Behandlung mit Acetanhydrid/Lutidin/1-methylimidazol in THF (1ml) blockiert. Der fertige Träger wird mit CH₂Cl₂ (3x1 ml) und Diethylether (3x1 ml) gewaschen und im Vakuum getrocknet. Die Beladung bezogen auf die eingeführte Monomethoxytritylfunktion beträgt 168mmolg⁻¹.

### Beispiel 4

### 6-((4-Methoxyphenyl)-diphenylmethylamino)-hex-1-yl succinylamidopropyl-Controlled-pore glass.

### (Mmt-hex-succ-CPG)

Die Herstellung erfolgt analog wie in Beispiel 3 beschrieben ausgehend von Aminopropyl-CPG (Firma Fluka) (550Å;1.0g;) und 6-((4-Methoxyphenyl)-diphenylmethylamino)-hex-1-yl hemisuccinat (48.7mg; 0.082mmol), 4-Ethylmorpholin (7.6ml) und TBTU (26.4mg; 0.082mmol) in DMF (3ml). Die Beladung des Mmt-hex-succCPG beträgt 91mmolg⁻¹

### Beispiel 5

### N-((4-Methoxyphenyl)-diphenylmethylamino)ethyl-N-((1-(N⁴-(4-tert-butylbenzoyl)-cytosyl)acetyl) glycin

### (Mmt-Aeg(c^{tBuBz})-OH)

1.63 g (2.28 mMol) N-((4-Methoxyphenyl)-diphenylmethylamino)ethyl-N-((1-(N⁴⁻(4-ter t-butylbenzoyl)-cytosyl)acetyl) glycin-methylester wurden in einer Mischung aus 10 ml Dioxan und 1 ml Wasser gelöst und bei 0°C unter Rühren tropfenweise mit 4.56 ml 1N NaOH versetzt. Nach 2h wurde durch tropfenweise Zugabe von 1N KHSO₄ der pH auf 5 gestellt, von ausgefallenen Salzen abfiltriert und mit wenig Dioxan nachgewaschen. Die vereinigten Filtrate werden im Vakuum eingedampft und der Rückstand zweimal mit Methanol und Dichlormethan koevaporiert. Das so erhaltene Rohprodukt wurde an Kieselgel mit einem Gradienten von 2-10% Methanol und 1 % Triethylamin in Dichlormethan chromatographisch gereinigt. Die das Produkt enthaltenden Fraktionen wurden vereinigt und im Vakuum eingeengt. Durch Koevaporation mit Pyridin und anschließend Toluol wurde noch vorhandenes überschüssiges Triethylamin entfernt. Man erhielt 0.831 g Produkt als fast weißen Schaum. Electrospray MS (Negativ-lon) 700.7 (M-H).
R_{f} 0.28 (CH₂Cl₂:MeOH/9:1), 0.63 (CH₂Cl₂:MeOH/7:3).

### Beispiel 6

### N-((4-Methoxyphenyl)-diphenylmethylamino)ethyl-N-((1-thyminyl)acetyl) glycin (Mmt-Aeg(t))-OH.

Das Produkt aus der obigen Reaktion wurde in einer Mischung aus10 ml Dioxan und 2 ml Wasser gelöst. Die Lösung wurde auf 0°C gekühlt und tropfenweise 1 N Natronlauge zugegeben bis ein pH-Wert von 11 erreicht war. Nach 2 h Reaktionszeit war die Reaktion beendet und die Lösung wurde durch vorsichtige Zugabe von 2 N KHSO₄-Lösung auf pH 5 gestellt Die Lösung wurde dreimal mit Ethylacetat extrahiert, die vereinigten organischen Phasen über Natriumsulfat getrocknet und im Vakuum eingeengt. Das so erhaltene Rohprodukt wurde an Kieselgel mit einem Gradienten von 5-10% Methanol und 1 % Triethylamin in Dichlormethan chromatographisch gereinigt. Die das Produkt enthaltenden Fraktionen wurden vereinigt und im Vakuum eingeengt. Durch Koevaporation mit Pyridin und anschließend Toluol wurde noch vorhandenes überschüssiges Triethylamin entfernt. Man erhielt 1.065 g Produkt als farblosen Schaum. Elektrospray MS (Negativ-lon) 1112.0 (2M-H)⁻, 555.3 (M-H)⁻
R_{f} 0.28 (CH₂Cl₂:MeOH/8:2)

### Beispiel 7

### N-((4-methoxyphenyl)-diphenylmethylamino)ethyl-N-(9-(N²-(isobutanoyl)-guanosyl)acetyl)glycin

### (Mmt-Aeg(g^{iBu})-OH)

N-((4-methoxyphenyl)-diphenylmethylamino)ethyl-N-(9-(N²-(isobutanoyl)-guanosyl)acetyl)glycinmethylester (1.15g; 1.72mmol) wird in Dioxan (10ml) gelöst und über einen Zeitraum von 2.5h bei 0°C tropfenweise mit 1M wäßriger Natronlauge (10.32ml) in 5 Teilen versetzt. Nach weiteren 2h Reaktionszeit bei Raumtemperatur wird die Lösung durch tropfenweise Zugabe von 2M wäßriger Kaliumhydrogensulfatlösung auf pH5 gestellt. Die ausgefallenen Salze werden abfiltriert und mit wenig Dioxan nachgewaschen.
Die vereinigten Filtrate werden im Vakuum zur Trockene eingedampft und der Rückstand je zweimal mit Ethanol sowie Dichlormethan:Methanol 1/1 koevaporiert. Die Reingung erfolgt durch Säulenchromatographie an Kieselgel durch Elution mit einem Gradienten von 10-20% Methanol in Dichlormethan (mit 1 % Triethylamin) . Man erhält das Produkt als weißen Schaum.
Ausbeute: 1.229g
ESMS (Negative-lon): 650.3(M-H)⁻
R_{f} 0.25 (Dichlormethan:Methanol/8:2)

### Beispiel 8

### N-((4-Methoxyphenyl)-diphenylmethylamino)ethyl-N-(9-(N⁶-(4-methoxybenzoyl)-adenosyl)acetyl) glycin

### (Mmt-Aeg(a^{MeoBz})-OH)

N-((4-methoxyphenyl)-diphenylmethylamino)ethyl-N-(9-(N⁶-(4-methoxybenzoyl)-adenosyl)acetyl) glycinmethyl ester (1.70g; 2.38mmol) wird in Dioxan (10ml) gelöst und über einen Zeitraum von 2.5h bei 0°C tropfenweise mit 1M wäßriger Natronlauge (10.32ml) in 5 Teilen versetzt. Nach weiteren 2h Reaktionszeit bei Raumtemperatur wird die Lösung durch tropfenweise Zugabe von 2M wäßriger Kaliumhydrogensulfatlösung auf pH5 gestellt. Die ausgefallenen Salze werden abfiltriert und mit wenig Dioxan nachgewaschen.
Die vereinigten Filtrate werden im Vakuum zur Trockene eingedampft und der Rückstand je zweimal mit Ethanol sowie Dichlormethan:Methanol 1/1 koevaporiert. Die Reinigung erfolgt durch Säulenchromatographie an Kieselgel durch Elution mit einem Gradienten von 10-20% Methanol in. Dichlormethan (mit 1% Triethylamin). Man erhält das Produkt als weißen Schaum.
Ausbeute: 1.619g
ESMS (Negative-lon): 698.3(M-H)⁻
R_{f} 0.10 (Dichlormethan:Methanol/8:2)

### Beispiel 9

### N-((4-Methoxyphenyl)-diphenylmethyloxy)ethyl-N-((1-thyminyl)acetyl) glycin (Mmt-Oeg(t)-OH)

0.5g (1.28mMol) N-((4-Methoxyphenyl)-diphenylmethyloxy)ethylglycin wurden in 10 ml DMF suspendiert und 0.47 ml (1.92 mMol) BSA wurden tropfenweise zugegeben. Nacheinander wurden dann 0.7 ml (5.1 mMol) Triethylamin und 0.26 g (1.28 mMol) Chlorcarboxymethylthymin zugefügt. Die Reaktionsmischung wurde 4 h bei Raumtemperatur gerührt, dann weitere 65 mg (0.32 mMol) Chlorcarboxymethylthymin zugegeben und noch für 16 h gerührt. Danach zog man das Lösungsmittel im Vakuum ab und reinigte das Rohprodukt an einer Kieselgelsäule mit einem Gradienten von 5-15% Methanol und 1 % Triethylamin in Dichlormethan. Die das Produkt enthaltenden Fraktionen wurden vereinigt und im Vakuum eingeengt. Das erhaltene bräunliche Öl wurde in wenig Dichlormethan gelöst und durch Zugabe von Diethylether das Produkt ausgefällt. Man erhielt das Produkt als fast weißes Pulver.
Ausbeute:0.219 g
Electrospray MS (Negativ lon) 556.3 (M-H)⁻
R_{f} 0.54 (CH₂Cl₂:MeOH/8:2).

### Beispiel 10

### 4-Nitrophenyl 4-(4, 4'-Dimethoxytrityloxy)-butyrat.

### Dmt-but-NPE

Das Natriumsalz der 4-Hydroxybuttersäure (1.26g; 10mmol) wird in wasserfreiem Pyridin (30ml) gelöst und mit 4,4'-Dimethoxytritylchlorid (3.39g; 3.05mmol) versetzt. Nach 16 Stunden gibt man 4-Nitrophenol (1.39g; 10mmol) und N,N'-Dicyclohexylcarbodiimid (2.06g; 10mmol) zu und rührt bei 22°C für weitere 48 Stunden. Der abgeschiedene Dicyclohexylharnstoff wird abfiltriert und mit Dichloromethan gewaschen. Das Filtrat wird konzentriert und der erhaltene Rückstand zweimal mit Toluol koevaporiert. Der Rückstand wird über eine Kieselgelsäule gereinigt (10-50% Ethylacetat und 1% Triethylamin in Petrolether). Die gewünschte Verbindung fällt in Form eines schwach gelblich gefärbten Öls an.
Ausbeute 2.694g.
MS (FAB, MeOH/NBA/LiCI) 534.2 (M+Li)⁺; 527.2 M⁺.
R_{f} 0.34 (Petrolether:Ethylacetate = 75:25).

### Beispiel 11

### H-Oprg(t)-OH

3.68 g Thyminylessigsäure werden in 20 ml trockenem DMF gelöst und 6.65 g TOTU und 2.77 ml Triethylamin zugegeben. Die Mischung wird 30 min bei Raumtemperatur nachgerührt und dann langsam zu einer Lösung bestehend aus 5.32 g (3-Hydroxypropyl)-glycin, 20 ml Wasser, 20 ml DMF und 5.54 ml Triethylamin zugetropft. Man rührt noch 1 h bei Raumtemperatur nach und engt dann am Rotationsverdampfer im Vakuum ein. Der Rückstand wird in Wasser aufgenommen, mit 1 N Salzsäure auf pH 1.5 gestellt und mit Ethylacetat extrahiert. Die wäßrige Phase wird mit gesättigter Natriumhydrogencarbonatlösung auf pH 5 gestellt und am Rotationsverdampfer eingeengt. Der Rückstand wird mit 250 ml Ethanol versetzt und das dabei ausgefallenen Natriumchlorid abgesaugt. Das Filtrat wird eingeengt und das Rohprodukt an Kieselgel mit Dichlormethan/Methanol/ Ethylacetat 10:2:1 unter Zusatz von 1 % Triethylamin gefolgt von Dichlormethan/Methanol/ Ethylacetat 10:4:1 unter Zusatz von 1% Triethylamin chromatographisch gereinigt. Die das Produkt enthaltenden Fraktionen werden vereinigt und am Rotationsverdampfer im Vakuum eingeengt.
Ausbeute: 3.2 g
R_{f} 0.15 (Dichlormethan/Methanol/ Ethylacetat 10:2:1 + 1% Triethylamin)
MS(ES⁺): 300.2(M+H)⁺.

### Beispiel 12

### Dmt-Oprg(t)-OH

3.2 g H-Oprg(t)-OH werden ind 40 ml DMF gelöst, 5.93 ml Triethylamin hinzugegeben und bei 0°C eine Lösung von 7.25 g Dmt-CI in 40 ml Dichlormethan innerhalb von 20 min zugetropft. Man rührt noch 2 h bei Raumtemperatur nach, filtriert dann das ausgefallene Triethylaminhydrochlorid ab und engt das Filtrat am Rotationsverdampfer im Vakuum ein. Der Rückstand wird in Dichlormethan aufgenommen, mit Wasser extrahiert, die organische Phase mit Natriumsulfat getrocknet und am Rotationsverdampfer im Vakuum eingeengt. Die Reinigung des Rohproduktes erfolgt an Kieselgel mit Dichlormethan/Methanol/ Ethylacetat 10:2:1 unter Zusatz von 1% Triethylamin. Die das Produkt enthaltenden Fraktionen werden vereinigt und am Rotationsverdampfer im Vakuum eingeengt.
Ausbeute: 3.46 g
R_{f} 0,28 (Dichlormethan/Methanol/ Ethylacetat 10:2:1 + 1% Triethylamin)
MS(ES⁺) 602.4(M+H)⁺.

### Beispiel 13

### H-Obg(t(-OH

2.76 g Thyminylessigsäure werden in 15 ml trockenem DMF gelöst und 4.92 g TOTU und 2.08 ml Triethylamin zugegeben. Die Mischung wird 30 min bei Raumtemperatur nachgerührt und dann langsam zu einer Lösung bestehend aus 4.41 g (4-Hydroxybutyl)-glycin, 10 ml Wasser, 10 ml DMF und 4.16 ml Triethylamin zugetropft. Man rührt noch 3 h bei Raumtemperatur nach und engt das. Gemisch am Rotationsverdampfer im Vakuum ein. Der Rückstand wird in Wasser aufgenommen, mit 1 N Salzsäure auf pH 1.5 gestellt und mit Ethylacetat extrahiert. Die wäßrige Phase wird mit gesättigter Natriumhydrogencarbonatlösung auf pH 5 gestellt und am Rotationsverdampfer eingeengt. Das Rohprodukt wird an Kieselgel mit Dichlormethan/Methanol/Ethylacetat 10:2:1 unter Zusatz von 1% Triethylamin chromatographisch gereinigt. Die das Produkt enthaltenden Fraktionen werden vereinigt und am Rotationsverdampfer im Vakuum eingeengt.
Ausbeute: 3.7 g
R_{f} 0.11 (Dichlormethan/Methanol/ Ethylacetat 10:2:1 + 1 % Triethylamin)
MS(ES⁺) 314.2(M+H)⁺.

### Beispiel 14

### Dmt-Obg(t)-OH

3.6 g H-Obg(t)-OH werden in 40 ml DMF gelöst, 9.5 ml Triethylamin hinzugegeben und bei 0°C eine Lösung von 15.4 g Dmt-CI in 40 ml Dichlormethan innerhalb von 15 min zugetropft. Man rührt noch 2 h bei Raumtemperatur nach gibt weitere 40 ml Dichlormethan hinzu, filtriert dann das ausgefallene Triethylaminhydrochlorid ab und engt das Filtrat am Rotationsverdampfer im Vakuum ein. Der Rückstand wird in Dichlormethan aufgenommen, mit Wasser extrahiert, die organische Phase mit Natriumsulfat getrocknet und am Rotationsverdampfer im Vakuum eingeengt. Die Reinigung des Rohproduktes erfolgt an Kieselgel mit Dichlormethan/Methanol/ Ethylacetat 15:1:1 unter Zusatz von 1% Triethylamin. Die das Produkt enthaltenden Fraktionen werden vereinigt und am Rotationsverdampfer im Vakuum eingeengt.
Ausbeute: 3.45 g
R_{f} 0,29 (Dichlormethan/Methanol/ Ethylacetat 10:2:1 + 1% Triethylamin)
MS(ES⁺ + LiCl) 622.3(M+Li)⁺.

### Beispiel 15

### H-Opeg(t)-OH

2.76 g Thyminylessigsäure werden in 15 ml trockenem DMF gelöst und 4.92 g TOTU und 2.08 ml Triethylamin zugegeben. Die Mischung wird 30 min bei Raumtemperatur nachgerührt und dann langsam zu einer Lösung bestehend aus 4.83 g (5-Hydroxypentyl)-glycin, 10 ml Wasser, 10 ml DMF und 4.16 ml Triethylamin zugetropft. Man rührt noch 3 h bei Raumtemperatur nach und engt das Gemisch am Rotationsverdampfer im Vakuum ein. Der Rückstand wird in Wasser aufgenommen, mit 1 N Salzsäure auf pH 1.5 gestellt und mit Ethylacetat extrahiert. Die wäßrige Phase wird mit gesättigter Natriumhydrogencarbonatlösung auf pH 5 gestellt und am Rotationsverdampfer eingeengt. Das Rohprodukt wird an Kieselgel mit Dichlormethan/Methanol/Ethylacetat 10:2:1 unter Zusatz von 1% Triethylamin chromatographisch gereinigt. Die das Produkt enthaltenden Fraktionen werden vereinigt und am Rotationsverdampfer im Vakuum eingeengt.
Ausbeute: 3.34 g
R_{f} 0.19 (Dichlormethan/Methanol/ Ethylacetat 10:2:1 + 1% Triethylamin)
MS(DCI) 328.2(M+H)⁺.

### Beispiel 16

### Dmt-Opeg(t)-OH

3.2 g H-Opeg(t)-OH werden ind 40 ml DMF gelöst, 6.77 ml Triethylamin hinzugegeben und bei 0°C eine Lösung von 9.94 g Dmt-CI in 40 ml Dichlormethan innerhalb von 15 min zugetropft. Man rührt noch 2 h bei Raumtemperatur nach gibt weitere 40 ml Dichlormethan hinzu, filtriert dann das ausgefallene Triethylaminhydrochlorid ab und engt das Filtrat am Rotationsverdampfer im Vakuum ein. Der Rückstand wird in Dichlormethan aufgenommen, mit Wasser extrahiert, die organische Phase mit Natriumsulfat getrocknet und am Rotationsverdampfer im Vakuum eingeengt. Die Reinigung des Rohproduktes erfolgt an Kieselgel mit Dichlormethan/Methanol/ Ethylacetat 15:1:1 unter Zusatz von 1% Triethylamin. Die das Produkt enthaltenden Fraktionen werden vereinigt und am Rotationsverdampfer im Vakuum eingeengt.
Ausbeute: 3.6 g
R_{f} 0,27 (Dichlormethan/Methanol/ Ethylacetat 10:2:1 + 1% Triethylamin)
MS(ES⁺ + LiCI): 636.4(M+Li)⁺.

### Beispiel 17

### 5'-ATC GTC GTA TT-(but)-agtc-hex

Die DNA-Sequenz ist in Großbuchstaben, die PNA-Sequenz in Kleinbuchstaben angezeigt (Beispiel für den Strukturtyp Xlla in Schema 1).
Die Synthese der PNA's erfolgt beispielsweise an einem Ecosyn D-300 DNA Synthesizer (Fa. Eppendorf/Biotronik, Maintal) oder einem ABI 380B DNA Synthesizer (Fa. Applied Biosystems, Weiterstadt). Die Synthese des DNA-Teils wird im Prinzip nach der Standard Phosphoramidit-Chemie und den kommerziell erhältlichen Synthesezyklen durchgeführt. Zur Synthese des PNA-Teils werden die Methoden der Peptid-Synthese, wie nachfolgend erläutert, an die DNA-Synthesezyklen angeglichen.

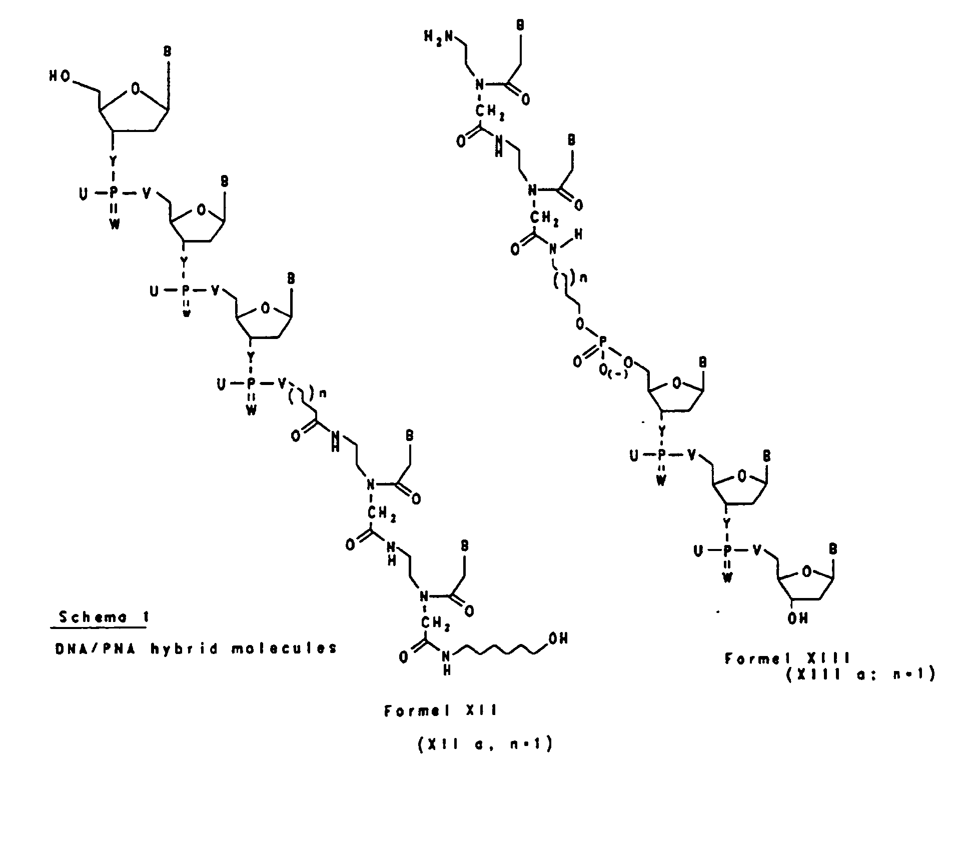

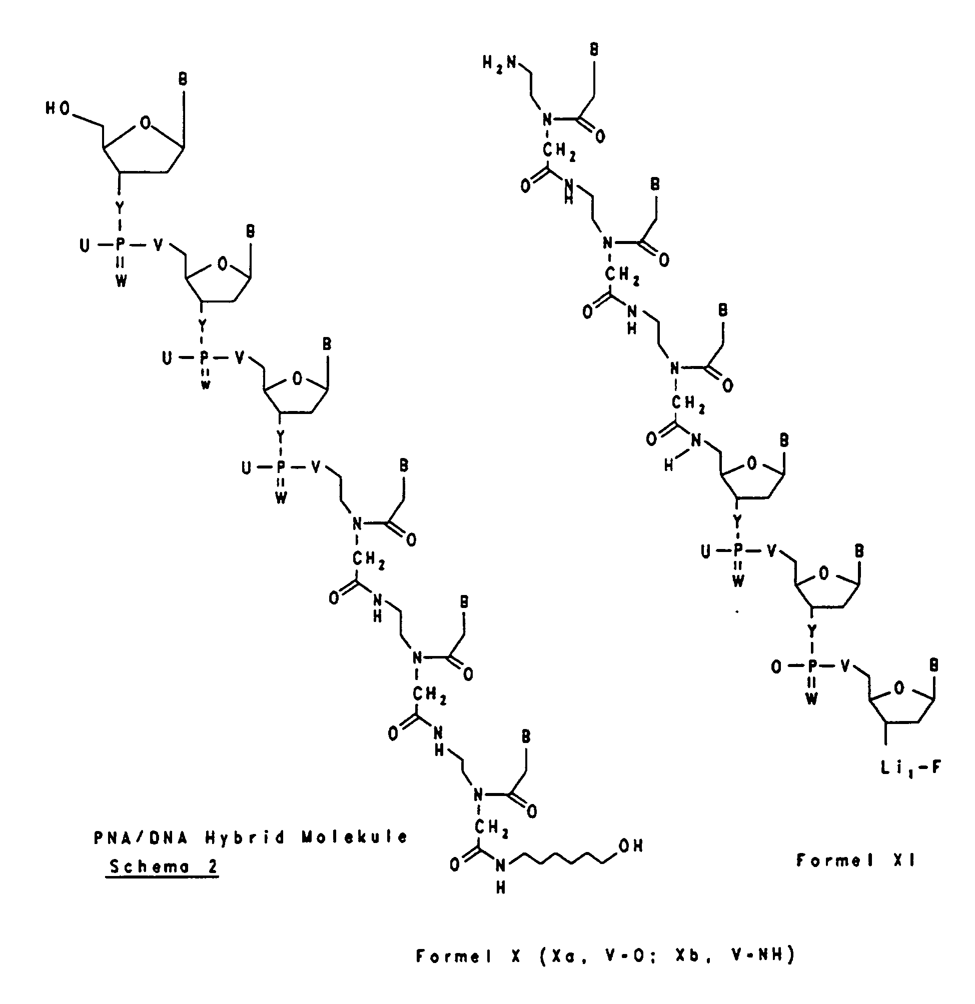

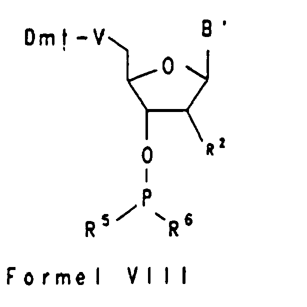

| | R⁵ | R⁶ | R² | V |
|---|---|---|---|---|
| VIII a | NC-CH₂CH₂-O- | -N(i-C₃H₇)₂ | H | O |
| VIII b | CH₃ | -N(i-C₃H₇)₂ | H | O |
| VIII c | C₆H₅ | -N(i-C₃H₇)₂ | H | O |
| VIII d | C₆H₅-C(O)-S(CH₂)₂-S | -N-pyrrolidin-1-yl | H | O |
| VIII e | NC-CH₂CH₂-O- | -N(i-C₃H₇)₂ | OCH₃ | O |
| VIII f | NC-CH₂CH₂-O- | -N(i-C₃H₇)₂ | H | NH |

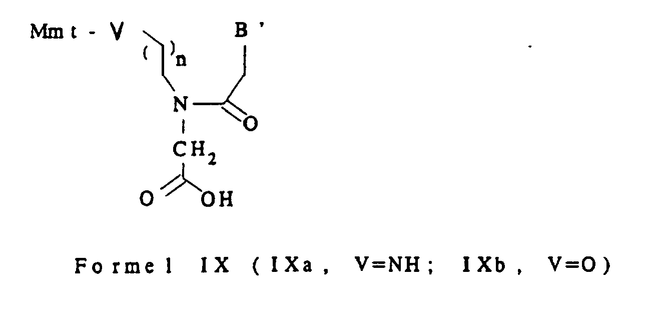

mit n = 1 - 8, bevorzugt 1 - 5, 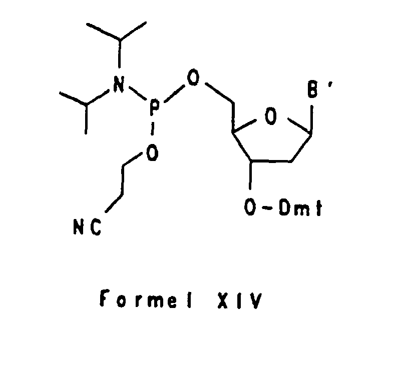 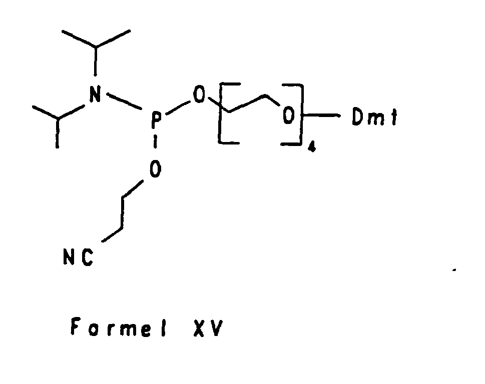 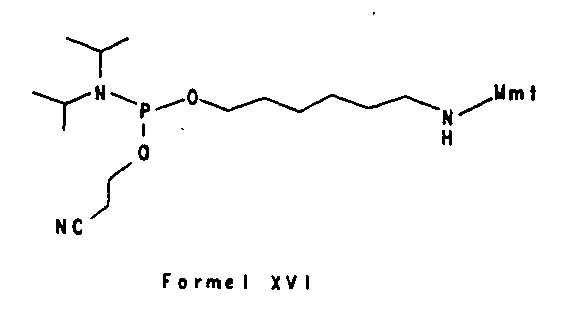 3 *µ*mol des mit Mmt-hex-succ beladenen CPG-Trägers (Beladung 91 *µ*mol /g) aus Beispiel 4 werden nacheinander mit folgenden Reagenzien behandelt:
Synthese des PNA-Teils (agtc-hex):
   1. Dichlormethan
   2. 3 % Trichloressigsäure in Dichlormethan
   3. Acetonitril abs.
   4. 3.5 M Lösung von 4-Ethylmorpholin in Acetonitril (Neutralisation)
   5. 0.4 M Lösung von (Mmt-Aeg(c^{tBuBz})-OH) aus Beispiel 5 in Acetonitril:DMF 9:1 / 0.9 M Lösung von ByBOP in Acetonitril / 3.5 M Lösung von 4-Ethylmorpholin in Acetonitril (10 Minuten Kupplungszeit).
   6. Schritt 5 wird viermal wiederholt.
   7. Acetonitril
      Die Schritte 1 bis 7, nachfolgend ein PNA-Reaktionszyklus genannt, werden zum Aufbau des PNA-Teils 3-mal wiederholt, wobei in Schritt 5 jeweils der laut Sequenz erforderliche Monomerbaustein aus den Beispielen 5 bis 8 eingesetzt wird.
Konjugation des Linkers (agtc-hex ---> (but)-agtc-hex ):
   8. Schritte 1 bis 4 von oben wiederholen
   9. 4-Nitrophenyl-4-(4, 4'-Dimethoxytrityloxy)-butyrat (105 mg) aus Beispiel 10 und Hydroxybenzotriazol (27 mg) in 2ml NEM in DMF für 15 Stunden
   10. Waschen mit DMF
   11. Waschen mit Acetonitril
   12. Dichlormethan
Synthese des DNA-Teils
   ((but)-agtc-hex) --> 5'-ATC GTC GTA TT-(but)-agtc-hex):
   13. Acetonitril abs.
   14. 3 % Trichloressigsäure in Dichlormethan
   15. Acetonitril abs.
   16. 10 µmol 5'-O-Dimethoxytritylthymidin-3'-phosphorigsäure-β-cyanoethylester-diisopropylamidit und 50 *µ*mol Tetrazol in 0.3 ml Acetonitril abs.
   17. Acetonitril
   18. 20 % Acetanhydrid in THF mit 40% Lutidinund 10 % Dimethylaminopyridin
   19. Acetonitril
   20. Jod (1.3 g in THF/Wasser/Pyridin; 70:20:5=v:v:v)

Die Schritte 13 bis 20, nachfolgend ein DNA-Reaktionszyklus genannt, werden zum Aufbau des Nucleotid-Teils 10-mal wiederholt, wobei im Schritt 16 jeweils das der Sequenz entsprechende 5'-0-Dimethoxytrityl(nucleobase)-3'-phosphorigsäure-β-cyanoeth ylester-diisopropylamidit eingesetzt wird.

Nach abgeschlossener Synthese erfolgt die Abspaltung der Dimethoxytritylgruppe wie in den Schritten 1 bis 3 beschrieben. Durch 1.5-stündige Behandlung mit Ammoniak wird das Oligomer vom Träger gespalten und zugleich werden die β-Cyanoethyl-Gruppen eliminiert. Zur Abspaltung der exozyklischen Amino-Schutzgruppen wird die ammoniakalische Lösung 5 Stunden bei 55°C gehalten. Vom erhaltenen Rohprodukt (325 OD₂₆₀)an 5'-ATC GTC GTA TT-(but)-agtc-hex werden 180 OD₂₈₀ durch Polyacrylamid-Gelelektrophorese gereinigt. Nach Entsalzung über eine Biogel^{R}-Säule (Fa. Biorad) erhält man davon 50 OD₂₆₀ hochreines Oligomer.

### Beispiel 18

### 5'-ATC GTC GTA TT-(Oeg(t))-agtc-hex

### (Beispiel für den Strukturtyp Xa in Schema 2; Erklärung für Oeg(t) vgl. Beispiel 9)

Die Synthese erfolgt analog wie in Beispiel 17 beschrieben, wobei jedoch im Schritt 9 anstelle des Dmt-buttersäure-p-nitrophenylesters der Linkerbaustein Mmt-Oeg(t)-OH aus Beispiel 9 unter den in Schritt 5 beschriebenen Bedingungen gekuppelt wird. Vom erhaltenen Rohprodukt (235 OD₂₆₀) an 5'-ATC GTC GTA TT-(Oeg(t))-agtc-hex werden 135 OD₂₆₀ durch Polyacrylamid-Gelelktrophorese gereinigt. Nach Entsalzung über eine BiogeI^{R}-Säule (Fa. Biorad) erhält man davon 20 OD₂₆₀ hochreines Oligomer.

### Beispiel 19

### N-ggg g(5'NH-C)T CₛCₛAₛ TGG GGₛGₛ T (Sequenz gegen HSV-1)

### (Beispiel für den Strukturtyp XI in Schema 2; ₛ bedeutet eine Phosphorothioat-Brücke; (5'NH-C) bedeutet einen 5'-Aminocytidylat-Rest; N gleich Aminoterminus)

Die Synthese efolgt ausgehend von einem CPG-Träger, welcher 5'-Dmtthymidin über dessen 3'-Ende gebunden hält. Es wird zunächst wie in Beispiel 17 beschrieben die Synthese des DNA-Teils durchgeführt (Schritte 13 bis 20), wobei im Falle der Phosphorothioat-Brücken (ₛ) die Oxidation im Schritt 20 mit Tetraethylthiuramdisulfid (TETD; User Bulletin No. 65 der Firma Applied Biosystems Inc.) erfolgt. Als Linkerbaustein wird ein Dmt-geschützter 5'-Amino-5'-desoxy-cytidylat-3'-phosphoramidit-Baustein der Formel Vlllf eingesetzt. Danach werden die PNA-Bausteine analog zu den Schritten 1 bis 7 in Beispiel 17 ankondensiert. Nach abgeschlossener Synthese wird das Oligomer durch 1.5-stündige Behandlung mit Ammoniak vom Träger gespalten und zugleich werden die β-Cyanoethyl-Gruppen eliminiert. Zur Abspaltung der exozyklischen Amino-Schutzgruppen wird die ammoniakalische Lösung 5 Stunden bei 55°C gehalten. Erst dann wird die Monomethoxytritylgruppe durch 2-stündige Behandlung mit 80 %-iger Essigsäure bei 22°C abgespalten. Das Produkt wird mittels Polyacrylamid-Gelelktrophorese gereinigt und über eine Biogel^{R}-Säule (Fa. Biorad) entsalzt.

### Beispiel 20

### 5'-G_{Me}G_{Me}G GCT CCA (Oeg(t))gg ggg t-hex

### (Beispiel für den Strukturtyp Xa in Schema 2; _{Me} bedeutet eine Methylphosphonat-Brücke; Erklärung für Oeg(t) vgl. Beispiel 9)

Die Synthese erfolgt analog wie in Beispiel 18 beschrieben, wobei aber zum Einbau der Methylphosphonatbrücken _{Me} die entsprechenden Methylphosphonat-Bausteine der Formel Vlllb im DNA-Reaktionszyklus eingesetzt werden.

### Beispiel 21

### 5'-C_{s,s}A_{s,s}C GT_{s,s}T GAG (but)Ggg cat-hex (c-myc antisense)

### (Beispiel für den Strukturtyp XIIa in Schema 1; _{s,s} bedeutet eine Phosphorodithioat-Brücke).

Die Synthese erfolgt analog wie in Beispiel 17 beschrieben, jedoch wird zum Einbau der Dithioat-Brücken der Baustein Vllld eingesetzt und and diesen Stellen die Oxidation (Schritt 20) mit TETD durchgeführt.

### Beispiel 22

### N-cga g(5'NH-A)A CAT CA (Oeg(t))ggt cg-hex(c-fos antisense)

### (5'NH-A bedeutet 5'-Amino-5'-desoxyadenylat; Erklärung für Oeg(t) vgl. Beispiel 9)

Die Synthese efolgt analog wie in Beispiel 18 beschrieben, wobei nach Abschluß der DNA-Synthese analog zu Beispiel 13 ein 5'-Aminonucleotid ankondensiert wird, welches die Konjugation des zweiten PNA-Teils erlaubt. Es werden also zuerst sechs PNA-Synthesezyklen durchgeführt und dann der Linkerbaustein aus Beispiel 9 aufgekuppelt. Danach werden sieben DNA-Synthesezyklen durchgeführt, wobei im letzten Zyklus der Baustein der Formel Vlllf eingesetzt wird. Nachdem noch vier PNA-Synthesezyklen durchgeführt wurden, wird die Abspaltung vom Träger und weitere Aufarbeitung wie in Beispiel 19 beschrieben vorgenommen.

### Beispiel 23

### F-cga g(5'NH-A)A CAT CAT GGT ₛCₛG-O-CH₂CH(OH)CH₂-O-C₁₆H₃₃

### (5'NH-A bedeutet 5'-Amino-5'-desoxyadenylat; F einen Fluorescein-Rest am Aminoterminus des PNA und ₛ eine Phosphorothioat-Brücke)

Die Synthese efolgt analog wie in Beispiel 19 beschrieben, jedoch ausgehend von einem CPG-Träger, der den Glycerol-hexadecylether gebunden hält. Nach Ausführung von 12 DNA-Synthesezyklen wird der Linkerbaustein Vlllf ankondensiert. Nachdem vier PNA-Synthesezyklen durchgeführt und die endständige Mmt-Gruppe abgespalten wurde, kann die freie Aminofunktion mit einem 30-fachen Überschuß an Fluoresceinisothiocyanat (FITC) quantitativ umgesetzt werden.

### Beispiel 24

### 3'-CCC TCT T-5'-(PEG)(PEG)-(Oeg(t))tg tgg g-hex

### (PEG bedeutet einen Tetraethylenglycolphosphatrest)

Die Synthese efolgt bezüglich des PNA-Teils analog wie in Beispiel 17 beschrieben. Nachdem sechs PNA-Einheiten ankondensiert wurden, wird das (Mmt-Oeg(t)-OH) aus Beispiel 9 aufgekuppelt. Als Linker wird dann wie im DNA-Synthesezyklus beschrieben zunächst zweimal das Tetraethylengylcolderivat der Formel XV ankondensiert, bevor die Synthese des DNA-Teils mit umgekehrter Orientierung (von 5' nach 3') durchgeführt wird. Dazu verwendet man in den DNA-Synthesezyklen anstelle der Nucleosid-3'-phosphoramidite im Schritt 16 jeweils die entspechenden Nucleosid-5'-phoshoramidite der Formel XIV, die kommerziell erhältlich sind. Die weitere Entschützung und Aufarbeitung erfolgt wie in Beispiel 17 beschrieben.

### Beispiel 25

### N-ccc tct t-(C6-link)(PEG)-3'-AAG AGG G-5'

### (PEG bedeutet einen Tetraethylenglycolphosphatrest; C6-link ist ein 6-Aminohexanolphosphatrest)

Die Synthese efolgt analog wie in Beispiel 17 (DNA-Synthesezyklus) beschrieben, jedoch ausgehend von einem CPG-Träger, der 3'-O-Dmt-Desoxyguanosin über eine 5'-O-Succinatgruppe gebunden hält. Nachdem sechs DNA-Einheiten mit Hilfe der Bausteine der Formel XIV ankondensiert wurden, wird als Linker zunächst einmal das Tetraethylengylcolderivat der Formel XV ankondensiert, bevor zur Einführung von C6-link das Phosphoramidit der Formel XVI gekuppelt wird. Danach wird der PNA-Teil wie in Beispiel 17 (PNA-Synthesezyklus) ansynthetisiert. Die weitere Entschützung und Aufarbeitung erfolgt wie in Beispiel 19 beschrieben.

### Beispiel 26

### 5'-TTT TTT TTT (but) ttt ttt-hex

Die Synthese erfolgt analog wie in Beispiel 17 beschrieben. Bevor das Produkt vom Träger gespalten und entschützt wird, entnimmt man eine Hälfte des trägergebundenen DNA/PNA-Hybrids zur Fluoreszenzmarkierung (Beispiel **27**). Die andere Hälfte wird wie in Beispiel 17 beschrieben entschützt und aufgearbeitet.

### Beispiel 27

### (FAM ist Fluorescein-Rest)

### 5'-FAM-TTT TTT TTT (but) ttt ttt-hex

Das trägergebundene DNA/PNA-Hybrid aus Beispiel 26 wird fluoreszenzmarkiert, indem die Schritte 13 bis 20 wie in Beispiel 17 beschrieben durchgeführt werden, wobei im Schritt 16 das Fluorescein-phosphoramidit der Fa. Applied Biosystems eingesetzt wird.

### Beispiel 28

### 5'-GGG GGG GGG (but) ttt ttt-hex

Die Synthese erfolgt analog wie in Beispiel 17 beschrieben. Bevor das Produkt vom Träger gespalten und entschützt wird, entnimmt man eine Hälfte des trägergebundenen DNA/PNA-Hybrids zur Fluoreszenzmarkierung (Beispiel 29). Die andere Hälfte wird wie in Beispiel 17 beschrieben entschützt und aufgearbeitet. Die Titelverbindung bindet als Triplex-forming Oligonucleotid mit hoher Affinität an einen DNA-Doppelstrang, der das Homopurin-Motiv 5'-AAA AAA GGG GGG GGG-3' enthält.

### Beispiel 29

### (FAM ist Fluorescein-Rest)

### 5'-FAM-GGG GGG GGG (but) ttt ttt-hex

Das trägergebundene DNA/PNA-Hybrid aus Beispiel 28 wird fluoreszenzmarkiert, indem die Schritte 13 bis 20 wie in Beispiel 17 beschrieben durchgeführt werden, wobei im Schritt 16 das Fluorescein-phosphoramidit der Fa. Applied Biosystems eingesetzt wird.

### Beispiel 30

### Biotin-C_{Phe}GpₕₑA GAA cat ca t(5'NH-G)G(Ome)U(Ome) C(Ome)G(Ome)-VitE (c-fos antisense)

### (N(Ome) bedeutet eine Nucleotideinheit N mit einer 2'-O-Methoxygruppe; _{Phe} bedeutet eine Phenylphosphonat-Brücke; 5'NH-G bedeutet 5'-Amino-5'-desoxyguanylat).

Die Synthese erfolgt analog wie in Beispiel 17 beschrieben ausgehend von CPG, welches mit Vitamin E beladen ist (MacKellar et al. (1992) Nucleic Acids Res, 20(13), 3411-17) und viermalige Kupplung des Bausteins der Formel Vllle nach dem DNA-Synthesezyklus. Nach Aufkupplung des 5'-Aminonucleotid-Bausteins der Formel Vlllf werden sechs PNA-Einheiten nach dem PNA-Synthesezyklus ankondensiert. Nach Neutralisation wird nach bekannter Methode das Phosphoramidit auf die Aminofunktion gekuppelt und der DNA-Synthesezyklus zum Aufbau des DNA-Teils entsprechend wiederholt, wobei im Falle der Phenylphosphonat-Brücken die Bausteine der Formel Vlllc in Schritt 16 eingesetzt werden. Zuletzt erfolgt die Aufkopplung der Endgruppe mit dem Biotin-Phsophoramididt der Fa. Glen Research. Nach abgeschlossener Synthese wird das Oligomer wie in Beispiel 19 beschrieben entschützt, wobei die Dimethoxytritylgruppe am Ende durch 2-stündige Behandlung mit 80 %-iger Essigsäure bei 22°C abgespalten wird.

### Beispiel 31

### A CAT CA (Oeg(t)) ggt cg-hex (c-fos antisense)

### (Erklärung für Oeg(t) vgl. Beispiel 9)

Die Synthese efolgt analog wie in Beispiel 18 beschrieben. Dabei werden zuerst fünf PNA-Synthesezyklen durchgeführt und dann der Linkerbaustein Oeg(t) aus Beispiel 9 aufgekuppelt. Danach werden sechs DNA-Synthesezyklen durchgeführt. Anschließend wird die Abspaltung vom Träger und die weitere Aufarbeitung wie in Beispiel 18 beschrieben vorgenommen.

### Beispiel 32

### A TAA TG (Oeg(t)) tct cg-hex (control oligomer for c-fos)

Die Synthese efolgt analog wie in Beispiel 18 beschrieben. Dabei werden zuerst fünf PNA-Synthesezyklen durchgeführt und dann der Linkerbaustein Oeg(t) aus Beispiel 9 aufgekuppelt. Danach werden sechs DNA-Synthesezyklen durchgeführt. Anschließend wird die Abspaltung vom Träger und die weitere Aufarbeitung wie in Beispiel 18 beschrieben vorgenommen.

### Beispiel 33

### a cat cat ggt cg-hex (c-fos antisense)

Dieses reine PNA-Oligomer wurde als Referenzverbindung analog wie in Beispiel 18 hergestellt, jedoch mit der Ausnahme, daß zwölf PNA-Cyclen durchgeführt wurden. Die Entschüztung der exozyklischen Amino-Schutzgruppen wird in ammoniakalischer Lösung (5 Stunden bei 55° C) durchgeführt. Erst dann wird die Monomethoxytritylgruppe durch 2-stündige Behandlung mit 80 %-iger Essigsäure bei 22° C abgespalten.

### Beispiel 34

### A (5-hexy-C)A(5-hexy-U) (5-hexy-C)A (Oeg(t)) ggt cg-hex (c-fos antisense) (Erklärung für Oeg(t) vgl. Beispiel 9; 5-hexy-C bedeutet 5-Hexinyl-cytidin, 5-Hexy-U bedeutet 5-Hexinyl-uridin)

Die Synthese efolgt analog wie in Beispiel 31 beschrieben, wobei jedoch anstelle der normalen Pyrimidin-Phosphoramidite die entsprechenden 5-Hexinylpyrimidin-nucleosid-phosphoramidite in die Kondensationsreaktion eingesetzt werden.

### Beispiel 35

### (FAM ist Fluorescein-Rest)

### 5'-FAM-TT (but) ttt ttt-hex

Die Synthese dieses PNA/DNA-Oligomers erfolgt analog wie in Beispiel 27 beschrieben, wobei allerdings nur zwei Thymidylat-Einheiten ankondensiert werden.

### Beispiel 36

### taa tac gac tca cta (5'HN-T)

### (5'HN-T bedeutet 5'-Amino-5'-desoxythymidin)

Dieses PNA/DNA-Oligomer, das aus 15 PNA-Einheiten und einer Nucleosid-Einheit aufgebaut ist, wurde als Primer für die DNA-Polymerase-Reaktion synthetisiert. Dabei geht man von einem Festphasen-Träger (Aminoalkyl-CPG) aus der das 5'-Monomethoxytritylamino-5'-deoxythymidin über dessen 3'-Hydroxygruppe als Succinat gebunden hält. Nach Abspaltung der Monomethoxytritylgruppe mit 3 % TCA in Dichlormethan werden 15 PNA-Cyclen wie in Beispiel 17 beschrieben durchgeführt. Die Entschüztung der exozyklischen Amino-Schutzgruppen wird in ammoniakalischer Lösung (5 Stunden bei 55° C) durchgeführt. Erst dann wird die Monomethoxytritylgruppe durch 2-stündige Behandlung mit 80 %-iger Essigsäure bei 22° C abgespalten. Man erhält ein PNA/DNA-Oligomer mit einer freien 3'-Hydroxygruppe, welche als Primer für eine DNA-Polymerase (Klenow) dient.

### Beispiel 37

### pₛ-rA(2'5')rA(2'5')rA(2'5')rA-spacer-(Oeg(t)tc ctc ctg cgg-hex

(pₛ bedeutet ein 5'Thiophopshat; spacer bedeutet ein Triethylenglycolphosphat; rA ist ein Riboadenylat; (2'5') bedeutet, daß die Internucleotidbindung von 2' nach 5' in der Ribose verläuft)
Die Synthese dieser Verbindung erfolgt analog wie in Beispiel 18 beschrieben, indem zunächst 14 PNA-Einheiten ankondensiert werden. Nachdem der Linkerbaustein Mmt-Oeg(t)-OH aus Beispiel 9 unter den in Schritt 5 beschriebenen Bedingungen eingeführt wurde, spaltet man die Mmt Gruppe mit 3 % TCA ab und führt den Spacer mit Hilfe des kommerziell erhältlichen Dmt-O-(CH₂CH₂O)₃-0-P(-OCH₂CH₂CN)N(i-C₃H₇)₃ Spacer-Phsophoramidits (Fa. Eurogentech; Brüssel) ein. Das (2'5')-verknüpfte Tetraadenylat wird wie in Beispiel 17 beschrieben mit Hilfe des käuflichen N⁶-Benzoyl-5'-O-Dmt-3'-O-tert-butyldimethylsilyl-adenosin-2'-O-cyanoethyl-di-isopropylamino-phosphoramidits (Fa. Milligen, Bedford, USA) ansynthetisiert, wobei die Kondensationszeit auf 2 x 5 Min. verlängert wird. Anstelle von Tetrazol wird in der Kupplungsreaktion der stärkere Aktivator 5-Ethylthiotetrazol eingesetzt. Nach Abspaltung der letzten Dmt-Gruppe wird das Oligomer mit Bis(β-cyanoetyloxy)-diisopropylaminophosphan an der 5'-OH-Gruppe phosphityliert. Nach Oxidation mit TETD und Entschützung mit Ammoniak und Desilylierung mit Fluorid erhält man die Titelverbindung, welche RNase L stimuliert.

### Beispiel 38

### pₛ-Co(2'5')Co(2'5')Co(2'5')Co-spacer-(Oeg(t))tc ctc ctg cgg-hex

### (pₛ bedeutet ein 5'Thiophosphat; spacer bedeutet ein Triethylenglycolphosphat; Co ist Cordycepin (3'-Deoxyadenosin); (2'5') bedeutet, daß die Internucleotidbindung von 2' nach 5' in verläuft)

Die Synthese wird analog wie in Beispiel 37 durchgeführt, jedoch wird anstelle des N⁶-Benzoyl-5'-O-Dmt-3'-O-tert-butyldimethylsilyl-adenosin-2'-O-cyanoethyl-di-isopropylamino-phosphoramidits das entsprechende N⁶-Benzoyl-5'-O-Dmt-cordycepin-2'-O-cyanoethyl-di-isopropylamino-phosphoramidit (Fa. Chemogen, Konstanz) eingesetzt und die Fluoridbehandlung entfällt.

### Beispiel 39

### 5'-GG GGG GGG (Oeg(t)) ttt ttt ttt-hex

Die Synthese erfolgt analog wie in Beispiel 18 beschrieben, wobei nach neun PNA-Kupplungen der Linkerbaustein Mmt-Oeg(t)-OH aus Beispiel 9 unter den in Schritt 5 beschriebenen Bedingungen ankondensiert wird, der die nachfolgende Kondensation von acht Guanylatresten erlaubt. Das resultierende PNA/DNA-Oligomer bindet mit hoher Affinität in antiparalleler Orientierung als Triplex-Forming-Oligonucleotid an doppelsträngige DNA, welche die Sequenz 5'..AAAAAAAAAAGGGGGGGG..3' aufweist.

### Beispiel 40

### Charakterisierung der PNA/DNA-Hybride

Die Charakterisierung erfolgt mit Hilfe der HPLC, Polyacrylamid-Gelelektrophorese (PAGE) und Negativionen Elektrospray Mässenspektrometrie (ES-MS⁻). Die Produkte werden wie oben beschrieben gereingt und zeigen danach bei der PAGE (20% Acrylamid, 2% Bisacrylamid und 7 M Harnstoff) eine einheitliche Bande. Die HPLC erfolgt auf Reversed Phase Säulen RP-18 der Fa. Merck (Eluent A:Wasser mit 0.1% TFA, B: Wasser/Acetonitril = 1:4; linearer Gradient) oder auf einer PA-100-Säule der Fa. Dionex (Eluent A: 20 mM NaOH and 20 mM NaCI; B:20 mM NaOH und 1.5 M NaCI; linearer Gradient). Für die ES-MS⁻ werden die PNA/DNA-Hybride durch Ammoniumacetat-Fällung oder andere Umsalzung in die Ammonium-Salze übergeführt. Die Probenaufgabe efolgt aus einer Lösung in Acetonitril/Wasser (1:1) mit 5 OD₂₆₀/ml Oligomer. Die Genauigkeit der Methode liegt bei ca. ± 1.5 Dalton.

### Beispiel 41

### Bestimmung der Zellaufnahme und Stabilität nach radioaktiver Markierung

### Radioaktive Markierung:

Eine allgemein anwendbare Markierung mit ³⁵S besteht darin, daß man bei der Synthese des DNA-Teils mindestens eine Oxidation im DNA-Synthesezyklus (Schritt 20 in Beispiel 17) mit elementarem ³⁵S durchführt. PNA/DNA-Hybride, die eine freie 5'-Hydroxygruppe haben, können mit Hilfe der Polynucleotid-Kinase nach an sich bekanneten Methoden mit ³²P oder ³⁵S markiert werden. PNA/DNA-Hybride, die eine freie 3'-Hydroxygruppe tragen, können nach bekannter Art mit 3'-terminaler Transferase markiert werden. Als Beispiel ist hier die 5'-Markierung des DNA-Teils wiedergegen: Das PNA/DNA-Hybrid mit einer freien 5'-Hydroxygruppe (500 pmol) aus Beispiel 17, 18 oder 26 wird in 425 *µ*l Wasser gelöst, diese Lösung auf 90°C erhitzt und abgeschreckt. Dann gibt man 50 *µ*l 10 x Kinase-Puffer und 50 *µ*l ³²P Gamma-ATP (6000 Ci/ mmol) bzw. ³⁵S-Gamma-ATP zu und inkubiert 1 Stunde bei 37°C. Die Reaktion wird durch Zugabe von 0.5 M EDTA-Lösung gestoppt. Die Entsalzung erfolgt mit Hilfe einer NAP^{R}-Säule der Firma Pharmacia.

### Bestimmung der Zellaufnahme:

Man inkubiert Vero-Zellen in 96-Napf Mikrotiterplatten in DMEM, 5 % FCS für 24 Stunden bei 37°C. Nachdem das Medium entfernt wurde, wäscht man die Zellen noch zweimal mit serumfreien DMEM. Das radioaktiv markierte Oligomer (10⁶cpm) wird mit nichtmarkiertem Oligomer auf eine Konzentration von 10 µM in Serum verdünnt und die Zellen bei 37°C damit inkubiert. Nach 1, 7 und 24 Stunden werden jeweils 150 *µ*l entnommen (Bezeichnung: "Überstand 1"). Die Zellen in den Näpfen der Mikrotiterplatten werden 7-mal mit 300 µl frischem Medium gewaschen und die vereinigten Waschmedien (Bezeichnung: "Überstand 2") im Szintillationszähler gemessen. Danach gibt man 100 µl Trypsinlösung zu, wartet 30 Sekunden und zieht den Überstand ab. Zur Ablösung der Zellen von der Platte inkubiert man 3 Min. bei 37°C. Die abgelösten Zellen werden in 1.5 ml Eppendorf-gefäße übergeführt und bei 2000 rpm für 6 Minuten zentrifugiert ("Überstand 3"). Die Überstände 1 (5*µ*l), 2 und 3 (0.5 ml) werden jeweils separat am Szintillationszähler gemessen. Daraus errechnet sich die Aufnahme des Oligomers in pmol per 100 000 Zellen, wobei Überstand 3 die zellgebundene Oligomerfraktion und die Summe der Überstände 1 und 2 die nicht zellgebundene Oligomerfraktion darstellen.

### Ergebnis:

| Inkubationszeit | Zellaufnahme | |
|---|---|---|
| in Stunden | in pmol Oligomer / 10⁵ Zellen PNA/DNA-Hybrid | DNA |
| 1 | 0.25 | 0.36 |
| 7 | 0.54 | 0.57 |
| 24 | 0.75 | 0.78 |

### Untersuchung der Stabiltät des Oligomers im Medium mit Zellen:

Der Überstand 1 (10 *µ*l) wird mit 5 *µ*l 80 % Formamid (mit Xylencyanol und Bromphenolblau) gemischt, auf 95°C erhitzt (5 Minuten) und auf ein Polyacrylamidgel (20 % Acrylamid, 7 M Harnstoff) geladen. Nach der Entwicklung des Gels im elektrischen Feld ordnet man die Banden auf dem Gel mittels Autoradiographie dem "Stabilen Oligomer" bzw. die Fehlbanden dem "abgebauten Oligomer" zu.

Das PNA/DNA-Oligomer aus Beispiel 26 ist nach 24 Stunden Inkubationszeit zu 69 % stabil; DNA-Oligomer zu 3 % stabil.

Das PNA/DNA-Oligomer aus Beispiel 31 besitzt unter diesen Bedingungen eine Halbwertszeit von 32 h, während das entsprechende DNA-Oligonucleotid eine Halbwertszeit von ca. 2 h aufweist.

### Beispiel 42

### Bestimmung der Zellaufnahme nach Fluoreszenz-Markierung:

Man läßt die COS-Zellen bis zur Konfluenz in Dulbecco's MEM, das mit 10 % FCS supplementiert wurde, in 5 cm Petrischalen heranwachsen. Die Zellen werden zweimal mit serumfreien DMEM gewaschen. Mit Hilfe einer sterilen Nadel wird eine Fläche von ca. 1 cm² in der Mitte der Petrischale eingekratzt. In diese Fläche wird die zu untersuchende PNA/DNA-Oligomerlösung (0.1 mM) aufgebracht. Es wird bei 37°C unter CO₂ Atmosphäre inkubiert. Nach 2, 4 und 16 Stunden werden die Zellen durch Fluoreszenzmikroskopie untersucht. Dazu werden die Zellen viermal mit serumfreien DMEM gewaschen, mit einem Glasträger abgedeckt und unter dem Fluoreszenzmikroskop bzw. durch Phasenkontrast bewertet. Als Vergleich zu den PNA/DNA-Hybridmolekülen wurde ein fluoreszenzmarkiertes PNA (ohne DNA-Teil) F-(but)-tttt ttt-hex untersucht. Nach zwei Stunden Inkubation der Zellen mit diesem PNA zeigen > 90 % der Zellen Anzeichen starker morphologischer Veränderungen und Zelltod. Die meisten Zellen weisen starke Vakuolisierung auf. Die Plasmamembran, das Cytosol und der Nucleus zeigen keine Aufnahme an PNA. Nach weiteren zwei Stunden Inkuabtion mit dem reinen PNA sind alle Zellen abgestorben. Anders verhält es sich mit den erfindungsgemäßen DNA/PNA-Oligomeren. Bereits nach zweistündiger Inkubation der Zellen mit den DNA/PNA-Oligomeren weisen die Zellen eine punktförmige intrazelluläre Verteilung der DNA/PNA-Oligomeren auf. Die Zellen erleiden auch nach längerer Inkubation keinen Zelltod.

### Beispiel 43

### Bestimmung der Schmelztemperaturen:

Die Bestimmung der Schmelztemperaturen erfolgen mit Hilfe eines HP 8452A Diodenarray-Spektrophotometers, eines HP 89090A Peltier-Elements und der HP Temperature Control Software Rev. B5.1 (Fa. Hewlett Packard). Es wird in 0.5°C/min. Schritten in 10mM HEPES und 140 mM NaCl (pH 7.5) als Puffer gemessen. Die Oligomerkonzentration beträgt 0.5 bis 1 OD₂₆₀ pro ml.

Ergebnis für das Produkt aus Beispiel 17 bzw. 18:

| | T_{M} gegen DNA |
|---|---|
| 5'-ATC GTC GTA T(Oeg(t)a gtc-hex | T_{M} = 51.5 °C |
| 3'-TAG CAG CAT A A T CAG-5' | antiparallel |
| 5'-ATC GTC GTA T(Oeg(t)a gtc-hex | T_{M} < 20°C |
| 5'-TAG CAG CAT A A T CAG-3' | parallel |
| 5'-ATC GTC GTA TT(but)a gtc-hex | T_{M} = 51.0 °C |
| 3'-TAG CAG CAT AA T CAG-5' | antiparallel |
| 5'-ATC GTC GTA TTA GTC-3' | T_{M} = 50.5 °C |
| 3'-TAG CAG CAT AAT CAG-5' | DNA·DNA antiparallel |
| 5'-ATC GTC GTA TT(but) a gtc-hex | T_{M} < 20°C |
| 5'-TAG CAG CAT AA T CAG-3' | parallel |

### Sequenz

| | | T_{M} | T_{M} |
|---|---|---|---|
| | | gegen DNA | gegen RNA |
| | | | (T=U) |
| 5'- ACA TCA TGG TCG -3' | DNA ap | 50.7°C | 48.6 °C |
| 3'- TGT AGT ACC AGC -5' | | | |
| 5'- ACA TCA tgg tcg -3' | (PNA-DNA) ap | 54.5°C | 54.7 °C |
| 3'- TGT AGT ACC AGC -5' | | | |
| 5'- ACA TCA tgg tcg -3' | (PNA-DNA) p | 20 °C | < 20 °C |
| 5'- TGT AGT ACC AGC -3' | | | |
| 5'- aca tca tgg tcg -3' | PNA ap | 58.8 °C | 66.6 °C |
| 3'- TGT AGT ACC AGC -5' | | | |
| 5'- aca tca tgg tcg -3' | PNA p | 46.3 °C | 44.8 °C |
| 5'- TGT AGT ACC AGC -3' | | | |
| 5'- ACA TCA TGG TCG -3' | S-DNA ap | 46.7 °C | 43.8 °C |
| 3'- TGT AGT ACC AGC -5' | | | |

TGG TCG bedeutet ein DNA-Teil, bei dem alle Internucleotid-Bindungen als Phosphorothioat vorliegen. Definition für p und ap s. Seite 5.

### Beispiel 44

### Testung auf antivirale Aktivität:

Die antivirale Aktivität der Prüfsubstanzen gegen verschiedene humanpathogene Herpesviren wird im Zellkulturtestsystem untersucht. Für den Versuch werden Affennierenzellen (Vero, 2x10⁵/ml) in serumhaltigem Dulbecco's MEM (5 % Fötales Kälberserum FCS) in 96-Napf-Mikrotiterplatten ausgesät und 24 h bei 37°C und 5 % CO₂ inkubiert. Das serumhaltige Medium wird dann abgesaugt und die Zellen werden zweimal mit serumfreiem Dulbecco's MEM (-FCS) überspült. Die Testsubstanzen werden in H₂O auf eine Konzentration von 600 *µ*M vorverdünnt und bei -18°C aufbewahrt. Für den Test erfolgen weitere Verdünnungsschritte in Dulbecco's Minimal Essential Medium (MEM). Je 100 *µ*l der einzelnen Prüfsubstanzverdünnungen werden zusammen mit 100 *µ*l serumfreiem Dulbecco's MEM (-FCS) zu den gespülten Zellen gegeben. Nach 3 h Inkubation bei 37°C und 5% CO₂ werden die Zellen mit Herpes simplex Virus Typ 1 (ATCC VR733, HSV-1 F-strain) oder mit Herpes simplex Virus Typ 2 (ATCC VR734, HSV-2 G-Strain) infiziert in Konzentrationen, bei denen der Zellrasen innerhalb von 3 Tagen vollkommen zerstört wird. Bei HSV-1 beträgt die Infektionsstärke 500 Plaque-bildende Einheiten (PFU) pro Napf, bei HSV-2 350 PFU/Napf. Die Versuchsansätze enthalten dann Prüfsubstanz in Konzentrationen von 80 *µ*M bis 0,04 *µ*M in MEM, ergänzt durch 100 U/ml Penicillin G und 100 mg/l Streptomycin. Alle Versuche werden als Doppelbestimmung durchgeführt mit Ausnahme der Kontrollen, die achtfach je Platte durchgeführt werden. Die Versuchsansätze werden 17 h bei 37°C und 5 % CO₂ inkubiert. Die Cytotoxizität der Prüfsubstanzen wird nach 20 h Gesamtinkubationszeit durch mikroskopische Begutachtung der Zellkulturen bestimmt. Als Dosis tolerata maxima (DTM) wird die höchste Präparatkonzentration bezeichnet, die unter den genannten Versuchsbedingungen noch keine mikroskopisch erkennbaren Zellschädigungen hervorruft. Es erfolgt daraufhin die Zugabe von FCS auf eine Endkonzentration von 4 % mit weiterer Inkubation für 55 h bei 37°C und 5% CO₂. Die unbehandelten Infektionskontrollen zeigen dann einen kompletten cytopathischen Effekt (CPE). Nach mikroskopischer Begutachtung der Zellkulturen werden diese dann mit Neutralrot entsprechend dem Vitalfärbungsverfahren nach Finter (1966) angefärbt. Die antivirale Aktivität einer Testsubstanz wird definiert als minimale Hemmkonzentration (MHK), die benötigt wird, um 30-60 % der Zellen vor dem virusbedingten cytopathogenen Effekt zu schützen. Die Aktivität der PNA/DNA-Chimären ist jeweils besser als die der entsprechenden DNA-Oligomeren oder PNA-Oligomeren.

### Beispiel 45

### Bestimmung der in vivo Aktivität: Inhibition der c-Fos Protein-Expression in der Ratte:

Die Bestimmung erfolgt wie beschrieben (Sandkühler et al. (1991) in : Proceedings of the Vlth World Congress on Pain, Charlton and Woolf, Editors; Elsevier, Amsterdam; Seite 313-318) durch Superfusion des Rückenmarks. Nach Laminektomy einer Barbiturat-anästhesierten Sprague-Dawley Ratte wird ein Zweikammer-Behältnis aus Silikon zur Aufnahme des Antisense Oligomers gebildet. Eine Kammer wird mit dem Antisense PNA/DNA-Derivat gefüllt, während die andere Kammer mit dem Kontroll Oligomer gefüllt wird (Konzentration je 75 *µ*M). Jeweils nach einer Stunde wird das Superfusat ausgetauscht. Nach 6 Stunden Superfusion wird die c-fos Expression durch Hitzebehandlung (52°C) der Hinterläufe stimuliert. Die Inhibiton der c-fos Expression kann immunohistochemisch an entsprechenden Gewebeschnittproben nachgewiesen werden. Das c-fos Antisense-Oligonucleotid aus Beispiel 31 bewirkt eine stärkere Inhibition der c-fos Expression als das entsprechende DNA-Oligonucleotid und das ensprechende PNA-Oligomer aus Beispiel 33.

### Beispiel 46

### RNase-H-Assay

Zur Bestimmung der RNase-H-Aktivität werden 1.3 OD des zu untersuchenden PNA/DNA-Oligomers mit 0.5 OD der komplementären RNA-Sequenz (TargetSequenz) in 50 *µ*l autoklaviertem, mit DEPC (Diethylpyrocarbonat) behandeltem Wasser, gelöst; 5 Minuten auf 80 °C erwärmt und anschließend binnen 15 Minuten auf 37 °C abgekühlt. Hierdurch werden zunächst beide Oligomere denaturiert, die nach Abkühlen in sequenzspezifischer Weise einen Nucleinsäure-Doppelstrang ausbilden.

Für den Test inkubiert man diesen RNA·PNA/DNA-Duplex mit 10 µlRNase H 10x Puffer, 1 *µ*l Dithiothreitol (DTT) und 2 *µ*l (entsprechend 10 u) RNase H der Firma USB. Der Inkubationsansatz wird mit autoklaviertem, DEPC behandeltem Wasser, auf das gewünschte Gesamtvolumen von 100 *µ*l vervollständigt. Die Proben werden bei 37 °C inkubiert. Für die kinetische Untersuchung wurden nach 0, 2 min, 10 min, und 1 h jeweils 20 *µ*l der Lösung abgenommen, für 5 Minuten auf 95 °C erhitzt und bei - 70 °C bis zur Analyse eingefroren. Die Untersuchung der RNase H-Spaltung der RNA erfolgt durch Gelelektrophorese. Es zeigte sich, daß PNA/DNA-Hybride, die Desoxyribonucleotid-Bausteine enthalten, die RNase H aktivieren, wobei der komplementäre RNA-Strang gespalten wird, während das PNA/DNA-Oligomer unversehrt aus der Reaktion hervorgeht. Die Spaltungsreaktion mit dem PNA/DNA-Oligomer verläuft etwas langsamer als mit einem entsprechenden Oligodeoxyribonucleotid gleicher Länge und Sequenz.

### Beispiel 47

### Herstellung eines HeLa-Zellextraktes mit RNase L-Aktivität

Um die Aktivität der zellulären Endoribonuklease L durch die 2'5'-Tetraadenylat-PNA/DNA-Konjugate zu stimulieren, wurde ein HeLa-Zellextrakt hergestellt. Dazu versetzt man 35 Flaschen mit je 20 ml Medium mit Dulbeccos MEM (minimal essential medium) und 10 % FCS (fötales Kälberserum). Die Ernte der Zellen kann nach Trypsinbehandlung vorgenommen werden. Nach Zentrifugation bei 1000 rpm werden 4 ml Zellernte erhalten. Diese füllt man zunächst mit 4 ml Wasser auf und gibt nach 3 Minuten 4 ml Puffer A (5,48 g HEPES; 15,5 g KCI; 2,488 g Mg-Acetat; 1232 *µ*l 2-Mercapto-ethanol ad 1 l Wasser) zu, um die Zellen zu lysieren. Anschließend zentrifugiert man die Lösung für 30 Minuten bei 0 °C in einer Ultrazentrifuge bei 30.000 rpm (ca. 100.000 g). Der Überstand von 8 ml Zellextrakt wird abgenommen und für die folgenden Untersuchungen bei - 20 °C eingelagert.

### Beispiel 48

### Untersuchung auf Aktivierung der RNase L

Für die Untersuchung dieses Extraktes auf die Endonuklease L werden zunächst 0.3 OD der RNA-Targetsequenz mit den jeweiligen PNA/DNA-Oligomeren 5 Minuten auf 80 °C erwärmt und anschließend zur Hybridisierung auf 37 °C abgekühlt. Der Duplex wird mit 20 *µ*l des Extraktes, 1.2 *µ*l Glycerin und RNase L-Puffer versetzt und bei 37 °C inkubiert. Das Gesamtvolumen beträgt anschließend 70 *µ*l. Für die kinetischen Untersuchungen werden Proben zu den Zeiten 0, 20 und 60 Minuten abpipettiert und zur Denaturierung der Enzyme 5 Minuten auf 95 °C erhitzt. Die Proben werden in einem Speedvac lyophilisiert und durch Gelelektrophorese analysiert. Die PNA-2'5'-Tetradenylat-Konjugate bzw. Tetracordycepin-Analoga aktivieren die zelluläre RNase L, während entsprechende Verbindungen ohne den Tetraadenylat-Teil die RNase L nicht stimulieren.

### Beispiel 49

### DNA-Polymerase-Reaktion

Als Templat für die DNA-Polymerase Reaktion dient folgendes 81-mer Oligodeoxynucleotid:

5′-GCC CCA GGG AGA AGG CAA CTG GAC CGA AGG CGC TTG TGG AGA
AGG AGT TCA TAG CTG GGC TCC CTA TAG TGA GTC GTA TTA-3′

### Die Sequenz des PNA/DNA-Primers ist:

### H-taa tac gac tca cta (5NH-T)-OH 3'.

Als Kontroll-Primer wird ein entsprechendes Oligodeoxynucleotid der Sequenz 5'- TAA TAC GAC TCA CTA TAG-3' eingesetzt.

Der Primer (0.15 nmol) und das Templat (0.15 nmol) in 5 *µ*l 10x PCR Puffer (500 mM KCI, 100 mM Tris-HCI, pH9, 1 % Triton X-100, 15 mM MgCl₂) werden mit 35 µl Wasser verdünnt und durch Erhitzen auf 95°C und Abkühlen hybridisiert. Dann gibt man 10 µl 2mM dNTP-Mischung (Nucleosid-5'-triphosphate) und 3 µl DNA-Polymerase (Klenow Fragment) zu und inkubiert je 0.5 Stunden bei 22 °C und 37 °C. Die Reaktionslösung wird danch auf einem 10 % Polyacrylamid-Gel (mit 1 % Bis) analysiert. Als Marker trägt man pBR322/Haelll-Verdau auf. Die Reaktion mit dem Kontroll-Primer zeigt ein doppelsträngiges DNA-Fragment mit der erwarteten Größe relativ zum Marker, während das Produkt aus dem PNA/DNA-Primer etwas schneller wandert. In beiden Fällen wandert in der Gelelektrophorese der Doppelstrang wesentlich schneller als der Templat-Einzelstrang.

## Patentansprüche

1. Polyamid-Oligonucleotid-Derivate der Formel lb, 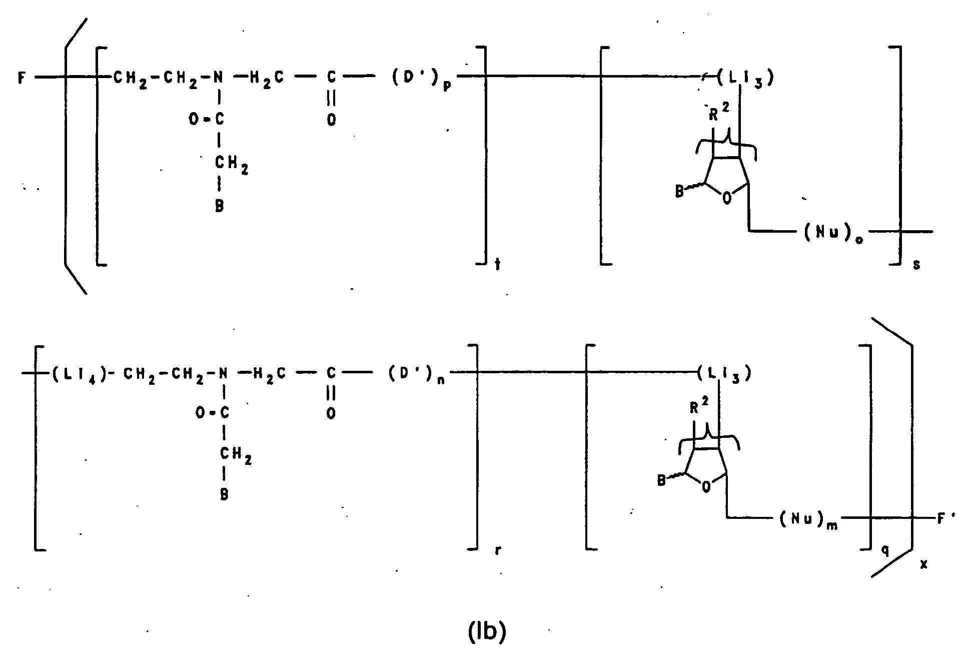 **dadurch gekennzeichnet, daß**
x 1 ist und
q = r = 1 und s = t = Null oder
r=s=1 und q= t =Null oder
q = r = s und t = Null sind;
R² Wasserstoff, Hydroxy, C₁-C₁₈-Alkoxy, Halogen, Azido oder Amino bedeutet;
B unabhängig voneinander für eine in der Nucleotidchemie übliche Base steht, und die "geschweifte Klammer" andeutet, daß sich R² und der benachbarte Substituent in 2'- und 3'-Stellung oder auch umgekehrt in 3'- und 2'-Stellung befinden können; und wobei die Polyamidstruktur mindestens eine Nukleobase enthält, die von Thymin verschieden ist;
Nu für einen Rest der Formeln lia oder IIb steht 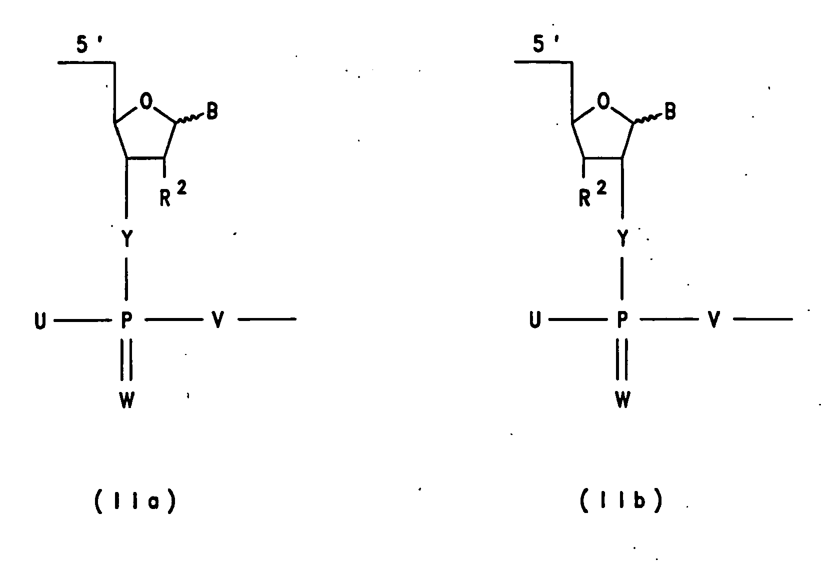 worin
R² und B wie oben definiert sind;
U Hydroxy, Mercapto, C₁-C₁₈-Alkyl, C₁-C₁₈-Alkoxy, C₆-C₂₀-Aryl, C₆-C₁₄₋Aryl-C₁-C₈-alkyl, NHR³ oder NR³R⁴ bedeutet und
R³ C₁-C₁₈-Alkyl oder C₁-C₄-Alkoxy-C₁-C₄-alkyl ist, und
R⁴ C₁-C₁₈-Alkyl ist oder
R³ und R⁴ zusammen mit dem sie tragenden Stickstoffatom einen 5-6-gliedrigen heterozyklischen Ring bedeutet, der zusätzlich ein weiteres Heteroatom aus der Reihe O, S, N enthalten kann;
V Oxy, Sulfanidyl oder Imino bedeutet;
W Oxo oder Thioxo bedeutet;
Y Oxy, Sulfanidyl, Methylen oder Imino bedeutet;
m = Null bis 20;
o = Null bis 20;
D' einen Rest der Formel IV bedeutet 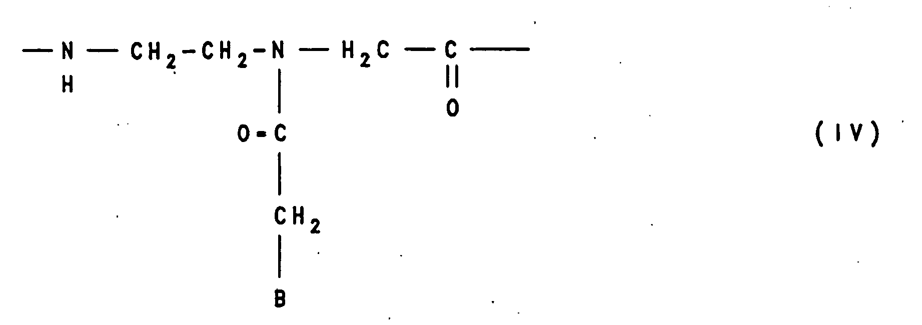 worin B wie oben definiert ist;
n = Null bis 20;
p = Null bis 20;
Li₃ und Li₄ unabhängig voneinander jeweils eine Struktur der Formel V
[(V')-(G)-(G')]_{ε} (V)
ist, wobei unabhängig voneinander
ε = 1 bis 5 ist,
V' Sauerstoff, NH, eine Bindung oder einen Rest der Formel VI 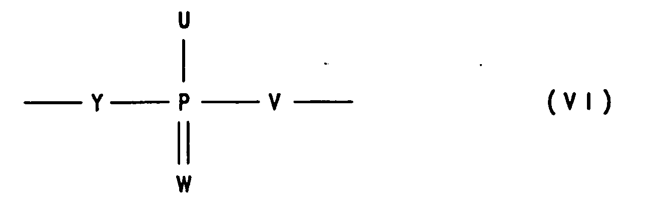
bedeuten,
worin U, V, W und Y wie oben definiert sind;
G C₁-C₁₂-Alkandiyl, wobei Alkandiyl gegebenenfalls durch Halogen, Amino, Hydroxy, C₁-C₁₈-Alkyl, C₁-C₁₈-Alkoxy, C₆-C₁₄-Aryl oder C₆-C₁₄-Aryl-C₁-C₁₈₋Alkyl substituiert sein kann; C₆-C₁₄-Aryl-di-C₁-C₁₂-Alkandiyl oder einer Gruppe der Formel (CH₂CH₂O)_{δ}CH₂CH₂, worin δ gleich 1 bis 11 sein kann; oder für eine Bindung stehen kann; und
G' Oxy, Sulfanidyl, Imino, -C(O)-, -C(O)NH-, eine Bindung oder einen Rest der Formel VI bedeuten, worin U, V, W und Y wie oben definiert sind; und
F und F' über eine Bindung verknüpft sind und/oder
F für R⁰-(A)ₖ-V- und
F' in Formel Ib für V¹ - (A)ₗ - R¹ stehen,
wobei
R⁰ Wasserstoff, C₁-C₁₈-Alkanoyl, C₁-C₁₈-Alkoxycarbonyl, C₃-C₈₋Cycloalkanoyl, C₇-C₁₅-Aroyl, C₃-C₁₃-Heteroaroyl oder eine Gruppe bedeuten, die die intrazelluläre Aufnahme des Oligomers begünstigt oder als Markierung einer DNA-Sonde dient oder bei der Hybridisierung des Oligomers an die target-Nucleinsäure diese unter Bindung, Quervernetzung oder Spaltung angreift; oder,
falls k = Null ist, R⁰ Wasserstoff ist oder zusammen mit V für einen Rest der Formel VII 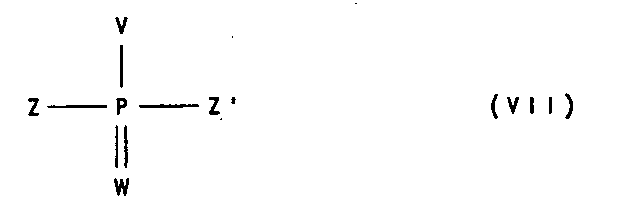 steht, worin
Z und Z' unabhängig voneinander Hydroxy, Mercapto, C₁-C₂₂-Alkoxy, C₁-C₁₈₋Alkyl, C₆-C₂₀-Aryl, C₆-C₁₄-Aryl-C₁-C₁₈-Alkyl, C₁-C₂₂-Alkylthio, NHR³, NR³R⁴, oder eine Gruppe bedeuten, die die intrazelluläre Aufnahme des Oligomers begünstigt oder als Markierung einer DNA-Sonde dient oder bei der Hybridisierung des Oligomers an die target-Nucleinsäure diese unter Bindung,
Quervernetzung oder Spaltung angreift, und worin
R³, R⁴, V und W wie oben definiert sind;
R¹Wasserstoff oder Q^{o}
wobei R¹ immer nur dann Wasserstoff ist,
wenn gleichzeitig I = Null und
in Fonnel Ib q = 1 oder q = r = Null und in F' = V¹ - (A)ₗ - R¹ mit V¹ = V bedeuten,
A den Rest einer natürlichen oder unnatürlichen Aminosäure bedeutet;
Q⁰ Hydroxy, OR', NH₂, NHR" bedeutet mit
R' = C₁-C₁₈-Alkyl und
R" = C₁-C₁₈-Alkyl, C₁-C₁₈-Aminoalkyl, C₁-C₁₈-Hydroxyalkyl;
V wie oben definiert ist;
V¹ eine Bindung oder V ist, wobei in F' nur in Formel lb mit q = Null und r = 1 V¹ immer für eine Bindung steht;
k Null bis 10 ist;
l Null bis 10 ist;
mit der Maßgabe, daß
a) falls in der Verbindung der Formel lb t = Null und s = 1 sind, steht Li₃ für eine Bindung;
b) falls in der Verbindung der Formel lb s = t = Null ist, steht Li₄ für eine Bindung;
wobei jedes Nucleotid in seiner D- bzw. L-Konfiguration vorliegen kann und sich die Base in α- oder β-Stellung befinden kann. '

2. Peptid-Oligonucleotid-Derivate der Formel lb gemäß Anspruch 1, **dadurch gekennzeichnet, daß** sich die Base in β-Stellung befindet.

3. Verfahren zur Herstellung von Peptid-Oligonucleotid-Derivaten gemäß den Ansprüchen 1 oder 2, **dadurch gekennzeichnet, daß** man
sukzessive eine PNA-Einheit bzw. DNA-Einheit mit jeweils einer Nucleobase an einen entsprechend derivatisierten Träger oder an eine wachsende Oligomerkette ankondensiert.

4. Peptid-Oligonucleotid-Derivate gemäß den Ansprüchen 1 oder 2 zur Anwendung als Heilmittel.

5. Peptid-Oligonucleotid-Derivate gemäß den Ansprüchen 1 oder 2 zur Anwendung als Heilmittel bei der Behandlung von Erkrankungen, die durch Viren hervorgerufen werden oder von Erkrankungen, die durch Integrine oder Zell-Zell-Adhäsionsrezeptoren beeinflußt werden, bei der Behandlung von Krebs oder bei der Verhinderung der Restenose.

6. Arzneimittel enthaltend ein Polyamid-Oligonucleotid-Derivat gemäß den Ansprüchen 1 oder 2.

7. Polyamid-Oligonucleotid-Derivate gemäß den Anprüchen 1 oder 2 zur Anwendung als Gensonde.

8. Polyamid-Oligonucleotid-Derivate gemäß den Ansprüchen 1 oder 2, **dadurch gekennzeichnet, daß** sich an mindestens einem Ende eine Nucleosid-Einheit mit einer 3'-Hydroxygruppe befindet, zur Anwendung als Primer.

9. Gensonden-Assay zur Bestimmung eines Oligo- bzw. eines Polynucleotid-Targets (RNA bzw. DNA), **dadurch gekennzeichnet, daß** man eine Gensonde nach Anspruch 7 in einem homogenen oder heterogenen Assay einsetzt.

10. Gensonden-Assay zur Bestimmung eines Oligo- bzw. eines Polynucleotid-Targets (RNA bzw. DNA), **dadurch gekennzeichnet, daß** man einen Primer nach Anspruch 8 einsetzt.

11. Gensonden-Assay nach den Ansprüchen 9 oder 10, **dadurch gekennzeichnet, daß** das Target durch Hybridisierung nach einer Targetamplifikation bestimmt wird.

## Claims

1. A polyamide-oligonucleotide derivative of the formula Ib 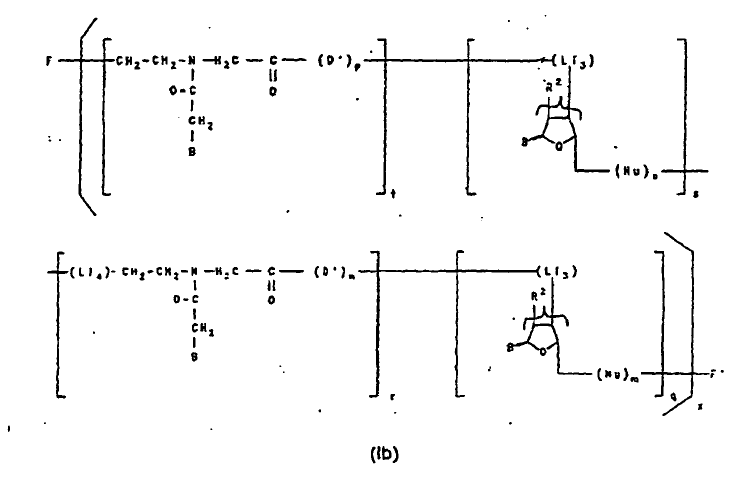 wherein
x is 1 and
q = r = 1 and s = t = zero or
r = s = 1 and q = t = zero or
q = r = s and t = zero;
R² is hydrogen, hydroxyl, C₁-C₁₈-alkoxy, halogen, azido or amino;
B is, independently of one another, a base customary in nucleotide chemistry, and the "curved bracket" indicates that R² and the adjacent substituent can be in the 2' position and 3' position or else conversely in the 3' position and 2' position, and in which the polyamide structure contains at least one nucleotide base which is different from thymine;
Nu is a radical of the formulae IIa or IIb 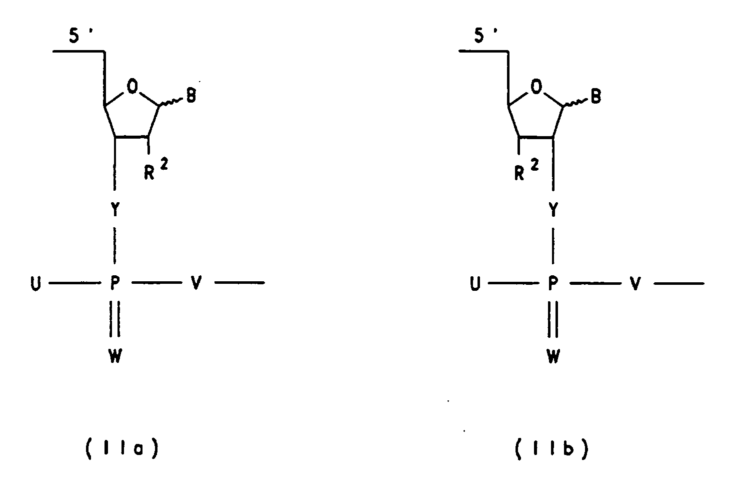 in which
R² and B are as defined above;
U is hydroxyl, mercapto, C₁-C₁₈-alkyl, C₁-C₁₈-alkoxy, C₆-C₂₀-aryl, C₆-C₁₄-aryl-C₁-C₈-alkyl, NHR³ or NR³R⁴, and
R³ is C₁-C₁₈-alkyl or C₁-C₄-alkoxy-C₁-C₄-alkyl, and
R⁴ is C₁-C₁₈-alkyl or
R³ and R⁴ is, together with the nitrogen atom carrying them, a 5-6-membered heterocyclic ring which can additionally contain another heteroatom from the series consisting of 0, S, N;
V is oxy, sulfanidyl or imino;
W is oxo or thioxo;
Y is oxy, sulfanidyl, methylene or imino;
m is zero to 20;
o is zero to 20;
D' is a radical of the formula IV 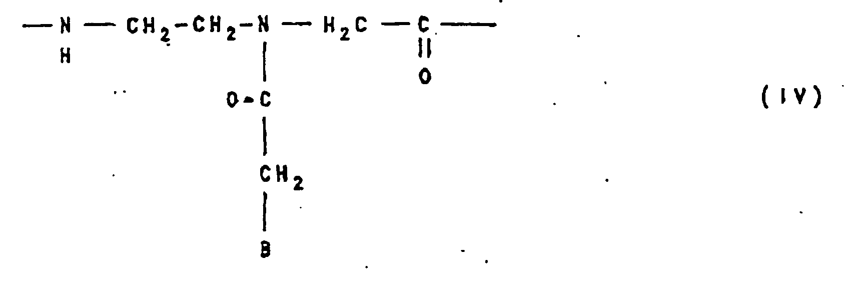
in which B is as defined above;
n is zero to 20;
p is zero to 20;
Li₃ and Li₄ are each, independently of one another, a structure of the formula V
[(V') - (G) - (G')]_{ε} (V)
where, independently of one another,
ε is 1 to 5,
V' is oxygen, NH, a bond or a radical of the formula VI 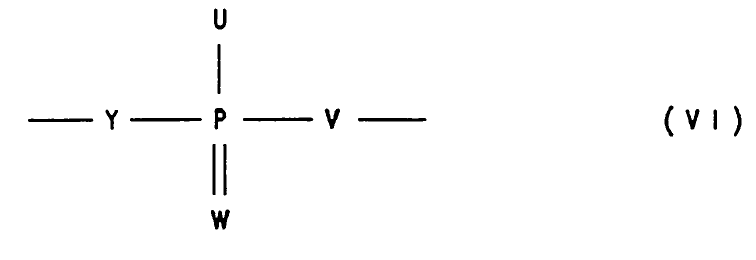 in which
U, V, W and Y are as defined above;
G can be C₁-C₁₂-alkanediyl, where alkanediyl can optionally be substituted by halogen, amino, hydroxyl, C₁-C₁₈-alkyl, C₁-C₁₈-alkoxy, C₆-C₁₄₋aryl, or C₆-C₁₄-aryl-C₁-C₁₈-alkyl C₆-C₁₄-aryl-di-C₁-C₁₂-alkanediyl, or a group of the formula (CH₂CH₂O)_{δ}CH₂CH₂ in which δ can be 1 to 11; or a bond; and
G' is oxy, sulfanidyl, imino, -C (O) -, -C (O) NH-, a bond or a radical of the formula VI in which U, V, W and Y are as defined above; and
F and F' are linked to one another by a bond and/or
F is R⁰ - (A)ₖ - V - and
F' in formula Ib is - V¹ - (A)₁ - R¹
where
R⁰ is hydrogen, C₁-C₁₈-alkanoyl, C₁-C₁₈₋alkoxycarbonyl, C₃-C₈-cycloalkanoyl, C₇-C₁₅₋aroyl, C₃-C₁₃-heteroaroyl or a group which favors intracellular uptake of the oligomer or serves as labeling of a DNA probe or, in the hybridization of the oligomer onto the target nucleic acid, attacks the latter with bonding, crosslinking or cleavage; or if k is zero, R⁰ is hydrogen or together with V is a radical of the formula VII 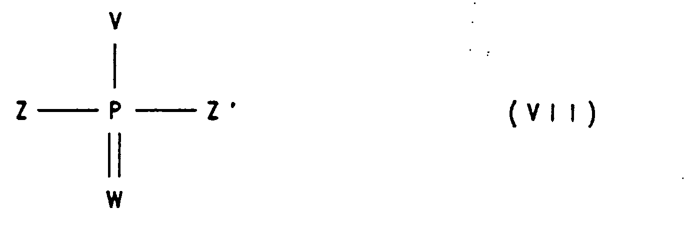 in which
Z and Z' are, independently of one another, hydroxyl, mercapto, C₁-C₂₂-alkoxy, C₁-C₁₈-alkyl, C₆-C₂₀-aryl, C₆-C₁₄-aryl-C₁-C₁₈-alkyl, C₁-C₂₂₋alkylthio, NHR³, NR³R⁴, or a group which favors intracellular uptake of the oligomer or serves as labeling of a DNA probe or, in the hybridization of the oligomer onto the target nucleic acid, attacks the latter with bonding, crosslinking or cleavage, and in which R³, R⁴, V and W are as defined above;
R¹ is hydrogen or Q^{o}
where R¹ is always only hydrogen
when at the same time 1 is zero and
in formula Ib q = 1 or q = r = zero and in F' = V¹ - (A) 1 - R¹ with V¹ - V,
A is the residue of a natural or unnatural amino acid;
Q^{o} is hydroxyl, OR', NH₂, NHR" with
R' = C₁-C₁₈-alkyl and
R" = C₁-C₁₈-alkyl, C₁-C₁₈-aminoalkyl, C₁-C₁₈₋hydroxyalkyl;
V is as defined above;
V¹ is a bond or is V, where V¹ is always a bond in F' only in formula Ib with q = zero and r = 1;
k is zero to 10;
l is zero to 10;
with the proviso that
a) if in the compound of the formula Ib t is zero and s is 1, Li₃ is a bond;
b) if in the compound of the formula Ib s = t = zero, Li₄ is a bond;
where each nucleotide can be in its D configuration or L configuration, and the base can be in the α or β position.

2. A peptide-oligonucleotide derivative of the formula Ib as claimed in claim 1, wherein the base is in the β position.

3. A process for the preparation of peptide-oligonucleotide derivatives as claimed in either of claims 1 and 2, which comprises successive condensation of a PNA unit or DNA unit with, in each case, one nucleotide base onto an appropriately derivatized support or onto a growing oligomer chain.

4. A peptide-oligonucleotide derivative as claimed in either of claims 1 and 2 for use as medicine.

5. A peptide-oligonucleotide derivative as claimed in either of claims 1 and 2 for use as medicine for the treatment of diseases caused by viruses or of diseases influenced by integrins or cell-cell adhesion receptors, for the treatment of cancer or for preventing restenosis.

6. A pharmaceutical containing a polyamide-oligonucleotide derivative as claimed in either of claims 1 and 2.

7. A polyamide-oligonucleotide derivative as claimed in either of claims 1 and 2 for use as gene probe.

8. A polyamide-oligonucleotide derivative as claimed in either of claims 1 and 2, wherein a nucleoside unit having a 3'-hydroxyl group is located on at least one end for use as primer.

9. A gene probe assay for the determination of an oligo- or polynucleotide target (RNA or DNA), wherein a gene probe as claimed in claim 7 is used in a homogeneous or heterogeneous assay.

10. A gene probe assay for the determination of an oligo- or polynucleotide target (RNA or DNA), wherein a primer as claimed in claim 8 is used.

11. A gene probe assay as claimed in either of claims 9 and 10, wherein the target is determined by hybridization after target amplification.

## Revendications

1. Dérivés de type polyamide-oligonucléotide de formule Ib, 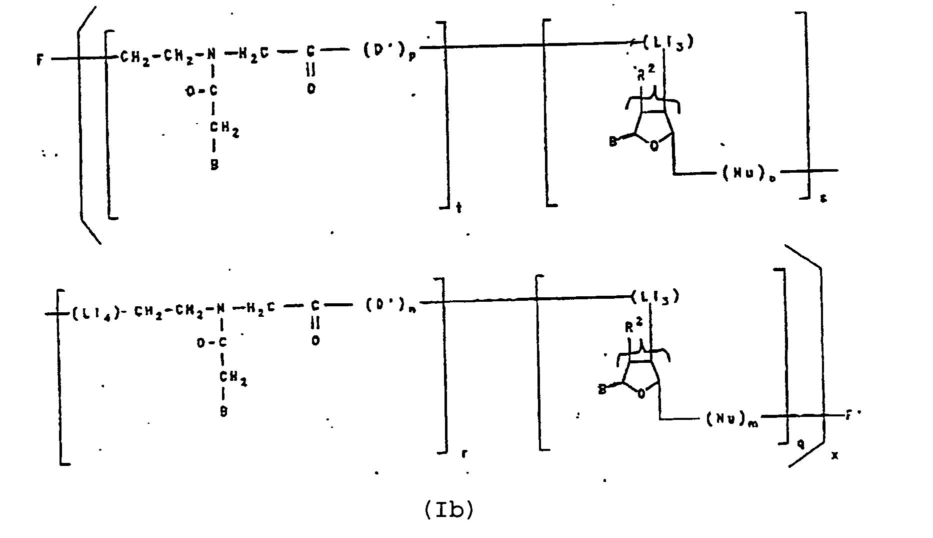 **caractérisés en ce que**
x est 1 et
q = r = 1 et s = t = zéro ou
r = s = 1 et q = t = zéro ou
q = r = s et t = zéro ;
R² représente un atome d'hydrogène ou d'halogène ou un groupe hydroxy, alcoxy en C₁-C₁₈, azido ou amino ;
B représente, chaque fois indépendamment, une base usuelle dans la chimie des nucléotides, et "l'accolade" signifie que R² et le substituant voisin peuvent se trouver en positions 2' et 3' ou bien inversement en positions 3' et 2' ; et la structure polyamide contenant au moins une base nucléique différente de la thymine ;
Nu représente un reste de formule IIa ou IIb 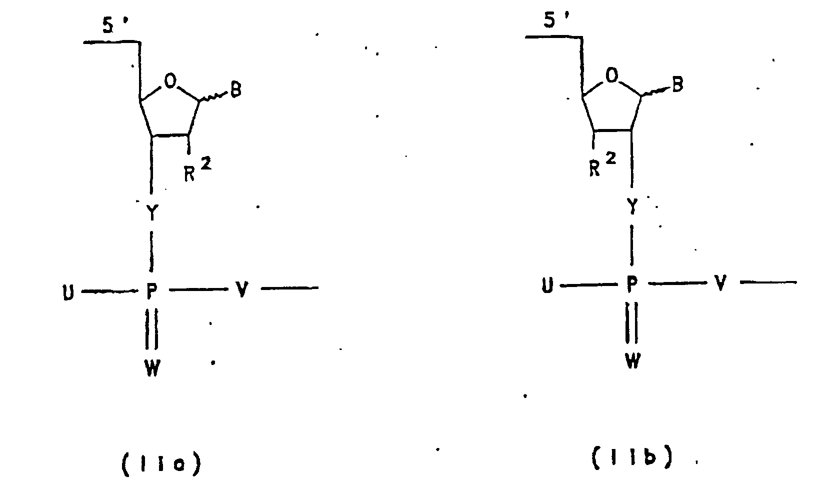 formules dans lesquelles
R² et B sont tels que définis ci-dessus ;
U représente un groupe hydroxy, mercapto, alkyle en C₁-C₁₈, alcoxy en C₁-C₁₈, aryle en C₆-C₂₀, aryl (C₆-C₁₄) -alkyle (C₁-C₈), NHR³ ou NR³R⁴ et
R³ est un groupe alkyle en C₁-C₁₈ ou alcoxy (C₁-C₄)-alkyle (C₁-C₄), et
R⁴ est un groupe alkyle en C₁-C₁₈ ou
R³ et R⁴ représentent ensemble avec l'atome d'azote qui les porte un cycle hétérocyclique à 5-6 chaînons, qui peut comporter en outre un autre hétéroatome choisi parmi O, S et N;
V représente un groupe oxy, sulfanidyle ou imino ;
W représente un groupe oxo ou thioxo ;
Y représente un groupe oxy, sulfanidyle, méthylène ou imino ;
m = zéro à 20 ;
o = zéro à 20;
D' représente un reste de formule IV 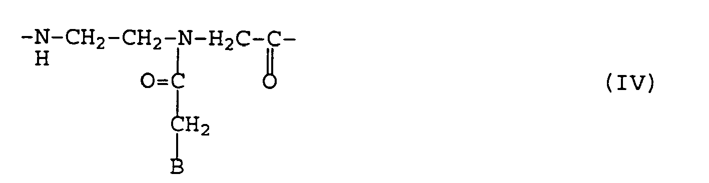 dans laquelle B est tel que défini plus
haut ;
n = zéro à 20 ;
p = zéro à 20 ;
Li₃ et Li₄ représentent chacun, indépendamment l'un de l'autre, une structure de formule V
[(V')-(G)-(G')]_{ε} (V)
dans laquelle, chaque fois indépendamment,
ε = 1 à 5,
V' représente un atome d'oxygène, NH, une liaison ou un reste de formule VI 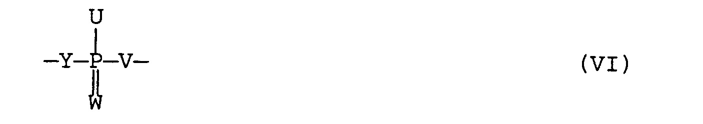 dans laquelle U, V, W et Y sont tels que définis plus haut ;
G représente un groupe alcanediyle en C₁-C₁₂, le groupe alcanediyle pouvant éventuellement être substitué par halogène, amino, hydroxy, alkyle en C₁-C₁₈, alcoxy en C₁-C₁₈, aryle en C₆-C₁₄ ou aryl (C₆-C₁₄) -alkyle (C₁-C₁₈) ; un groupe aryl(C₆-C₁₄)-dialcane(C₁-C₁₂)diyle ou un groupe de formule (CH₂CH₂O)_{δ}CH₂CH₂, dans lequel δ peut aller de 1 à 11 ; ou peut représenter une liaison ; et
G' représente un groupe oxy, sulfanidyle, imino, -C (O) -, -C (O)NH-, une liaison ou un radical de formule VI, U, V, W et Y étant tels que définis plus haut ; et
F et F' sont liés en une liaison et/ou
F représente R⁰-(A)ₖ-V- et
F' dans la formule Ib représente V¹-(A)₁-R¹,
où
R⁰ représente un atome d'hydrogène, un groupe alcanoyle en C₁-C₁₈, alcoxy (C₁-C₁₈) carbonyle, cycloalcanoyle en C₃-C₈, aroyle en C₇-C₁₅, hétéroaroyle en C₃-C₁₃ ou un groupe qui favorise la capture intracellulaire de l'oligomère ou sert de marqueur d'une sonde d'ADN ou, lors de l'hybridation de l'oligomère avec l'acide nucléique cible, accroche ce dernier par fixation, pontage ou coupure ; ou
lorsque k = zéro, R⁰ représente un atome d'hydrogène ou, conjointement avec V, un radical de formule VII 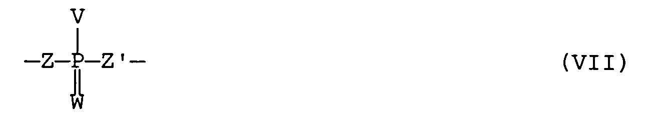 dans laquelle
Z et Z' représentent, indépendamment l'un de l'autre, un groupe hydroxy, mercapto, alcoxy en C₁-C₂₂, alkyle en C₁-C₁₈, aryle en C₆-C₂₀, aryl (C₆-C₁₄) -alkyle (C₁-C₁₈), alkyl (C₁-C₂₂) thio, NHR³, NR³R⁴ ou un groupe qui favorise la capture intracellulaire de l'oligomère ou sert de marqueur d'une sonde d'ADN ou, lors de l'hybridation de l'oligomère avec l'acide nucléique cible, accroche ce dernier par fixation, pontage ou coupure ; et où
R³, R⁴, V et W sont tels que définis plus haut;
R1 représente un atome d'hydrogène ou Q°
R¹ n'étant toujours un atome d'hydrogène que lorsque simultanément 1 = zéro et
dans la formule Ib q = 1 ou q = r = zéro et F' = V¹- (A) ₁-R¹ où V¹ = V,
A représente le reste d'un aminoacide naturel ou non naturel ;
Q⁰ représente un groupe hydroxy, OR', NH₂, NHR" où
R' représente un groupe alkyle en C₁-C₁₈ et
R" représente un groupe alkyle en C₁-C₁₈, aminoalkyle(C₁-C₁₈), hydroxyalkyle (C₁-C₁₈) ;
V est tel que défini plus haut;
V¹ est une liaison ou V, dans F' V¹ ne représentant toujours une liaison que dans la formule Ib où q = zéro et r = 1 ;
k est zéro à 10 ;
l est zéro à 10 ;
étant entendu que
a) lorsque dans le composé de formule Ib t = 0 et s = 1, Li₃ représente une liaison ;
b) lorsque dans le composé de formule Ib s = t = 0, Li₄ représente une liaison ;
chaque nucléotide pouvant se trouver en configuration D ou L et la base pouvant se trouver en position α ou β.

2. Dérivés de type polyamide-oligonucléotide de formule Ib selon la revendication 1, **caractérisés en ce que** la base se trouve en position β.

3. Procédé pour la préparation de dérivés de type polyamide-oligonucléotide selon la revendication 1 ou 2, **caractérisé en ce qu'**on condense successivement une unité de PNA ou une unité d'ADN comportant chacune une base nucléique avec un support convenablement fonctionnalisé ou une chaîne oligomère en croissance.

4. Dérivés de type polyamide-oligonucléotide selon la revendication 1 ou 2, pour utilisation en tant que médicamer,

5. Dérivés de type polyamide-oligonucléotide selon la revendication 1 ou 2, pour utilisation en tant que médicament dans le traitement de maladies qui sont provoquées par des virus ou de maladies qui sont sous l'influence d'intégrines ou de récepteurs d'adhésion cellule-cellule, dans le traitement du cancer ou dans l'empêchement de la resténose.

6. Médicament contenant un dérivé de type polyamide-oligonucléotide selon la revendication 1 ou 2.

7. Dérivés de type polyamide-oligonucléotide selon la revendication 1 ou 2, pour utilisation en tant que sonde génique.

8. Dérivés de type polyamide-oligonucléotide selon la revendication 1 ou 2, **caractérisés en ce qu'**à au moins une extrémité se trouve une unité nucléosidique comportant un groupe 3'hydroxy, pour utilisation en tant qu'amorce.

9. Essai à l'aide de sondes géniques, pour la détermination d'une cible oligonucléotidique ou polynucléotidique (ARN ou ADN), **caractérisé en ce qu'**on utilise une sonde génique selon la revendication 7 dans un essai homogène ou hétérogène.

10. Essai à l'aide de sondes géniques, pour la détermination d'une cible oligonucléotidique ou polynucléotidique (ARN ou ADN), **caractérisé en ce qu'**on utilise une amorce selon la revendication 8.

11. Essai à l'aide de sondes géniques selon la revendication 9 ou 10, **caractérisé en ce que** la cible est déterminée par hybridation après une amplification de la cible.
